(19) **Europäisches Patentamt**
**European Patent Office**
**Office européen des brevets**

(11) **EP 3 094 966 B1**

(12) **EUROPEAN PATENT SPECIFICATION**

(45) Date of publication and mention
of the grant of the patent:
**18.07.2018 Bulletin 2018/29**

(51) Int Cl.:
*G01N 29/02* (2006.01)    *G01N 29/036* (2006.01)
*G01N 29/44* (2006.01)    *G01N 11/16* (2006.01)

(21) Application number: **15703231.9**

(22) Date of filing: **19.01.2015**

(86) International application number:
**PCT/EP2015/050903**

(87) International publication number:
**WO 2015/107200 (23.07.2015 Gazette 2015/29)**

(54) **RESONATOR-BASED EVALUATION OF FLUID VISCOELASTIC PROPERTIES**

RESONATORBASIERTE AUSWERTUNG DER VISKOELASTISCHEN EIGENSCHAFTEN EINER FLÜSSIGKEIT

EVALUATION À BASE DE RÉSONATEUR DES PROPRIÉTÉS VISCOÉLASTIQUES D'UN FLUIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.01.2014 GB 201400812**

(43) Date of publication of application:
**23.11.2016 Bulletin 2016/47**

(73) Proprietor: **Dublin City University**
**Dublin D9 (IE)**

(72) Inventors:
• **KILLARD, Anthony J.**
**Bristol BS7 0LL (GB)**
• **EFREMOV, Vitaly**
**Dublin 9 (IE)**
• **LAKSHMANAN, Ramji Sitaraman**
**Dublin 9 (IE)**

(74) Representative: **Johnson, Richard Alan et al**
**Mewburn Ellis LLP**
**City Tower**
**40 Basinghall Street**
**London EC2V 5DE (GB)**

(56) References cited:
**US-A- 4 862 384     US-A1- 2011 129 929**

• **LAKSHMANAN RAMJI S ET AL: "Measurement of the evolution of rigid and viscoelastic mass contributions from fibrin network formation during plasma coagulation using quartz crystal microbalance", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, vol. 192, 28 October 2013 (2013-10-28), pages 23-28, XP028806887, ISSN: 0925-4005, DOI: 10.1016/J.SNB.2013.10.081**
• **MÜLLER LOTHAR ET AL: "Investigation of prothrombin time in human whole-blood samples with a quartz crystal biosensor", ANALYTICAL CHEMISTRY, AMERICAN CHEMICAL SOCIETY, vol. 82, no. 2, 15 January 2010 (2010-01-15), pages 658-663, XP007916036, ISSN: 0003-2700, DOI: 10.1021/AC9021117 [retrieved on 2009-12-14]**
• **KEIJI KANAZAWA K ET AL: "The oscillation frequency of a quartz resonator in contact with liquid", ANALYTICA CHIMICA ACTA, ELSEVIER, AMSTERDAM, NL, vol. 175, 1 January 1985 (1985-01-01), pages 99-105, XP002604127, ISSN: 0003-2670, DOI: 10.1016/S0003-2670(00)82721-X [retrieved on 2002-01-02]**

EP 3 094 966 B1

**Description**

FIELD OF THE INVENTION

The invention relates to a method of providing information on the mechanical characteristics of viscoelastic material in physical communication with, i.e. in contact with or coupled to, an oscillating resonator. In particular, the invention may relate to ascertaining properties of particles or films coupled from a fluid to the oscillator. For example, the invention may permit evaluation of qualitative and semiquantitative changes in blood or blood component viscoelasticity.

BACKGROUND TO THE INVENTION

Blood coagulation is a complex process involving several biochemical reactions, the end-point of which is the formation of a clot. A clot is a gel-like network formed at a site of injury and functions as a plug to prevent loss of blood. A global coagulation test looks at the entire cascade of reactions leading to a clot. Every clot that forms, results in a unique set of viscoelastic property changes depending on several factors that contribute to the coagulation cascade. A resonator's vibration characteristics are known to be dependent on viscoelastic properties of material it comes in contact with. A mechanical resonator is device that naturally oscillates at a certain frequency, with significantly larger amplitudes, known as the resonant frequency. The resonant frequency changes depending on the physical properties of the medium surrounding the resonator, i.e. in physical contact or in close proximity. For example, a small amount of mass (<< mass of resonator) attaching to the resonator results in a mass-dependent decrease in the resonant frequency. In contact with a liquid, the resonant characteristics are affected in two ways: a change in frequency and a significant reduction in oscillation amplitudes due to energy lost in maintaining the oscillations in a viscous medium.

It is known to use an oscillating resonator sensor (e.g. a quartz crystal microbalance (QCM)) to study mechanical properties of the physical relationship between viscous fluid and/or viscoelastic particles within a viscous fluid and a particular type of surface [1] that is in contact or close proximity to the fluid or particles. One surface of the resonator is brought in contact with the analyte liquid. The resonator surface can be gold, silicon dioxide or coated with materials to achieve different properties such as hydrophobic, hydrophilic, charged, etc. An AC signal is applied to a piezoelectric resonator and the local maximum of the conductance spectrum is determined. The piezoelectric resonator sensor is excited at one of its resonant frequencies, and two properties of its response are measured using an impedance analyser: (i) resonance frequency $f$ and (ii) half-bandwidth of resonance peak curve $\Gamma$.

Mechanical properties of a fluid such as viscosity and/or density as well as mechanical properties of a layer formed by particles interacting with (e.g. coupled or adhered to) the resonator, such as mass density and/or complex shear modulus, are typically estimated using shifts of both $f$ and $\Gamma$ values with respect to their values ($f_0$, $\Gamma_0$) when the sensor is unloaded. The shifts may be represented by the time-courses $\Delta f = f(t) - f_0$ and $\Delta\Gamma = \Gamma(t) - \Gamma_0$. It is possible to measure Q-factor ($Q$) or dissipation value ($D$) as alternatives to $\Gamma$ [2,3]. These parameters can be converted from one to another using the simple expression

$$D = \frac{1}{Q} = \frac{2\Gamma}{f_0}$$

An experiment to assess mechanical coupling or adhesion of a film to an oscillating (resonator) surface usually consists of three phases:

1. Measurement of $f_0$ and $\Gamma_0$ values before the resonator sensor comes in contact with a fluid to be investigated;
2. Measurement of values for $\Delta f(t_z)$ and $\Delta\Gamma(t_z)$ at a time $t_z$ after immersing the sensor in the fluid but before particles being to interact with it (e.g. adhere to it); and
3. Measurements of values for $\Delta f(t)$ and $\Delta\Gamma(t)$ at time $t$ after $t_z$, when coupling of viscoelastic material from the viscous liquid has occurred.

At step 2 it is possible to apply the Kanazawa equation to define fluid viscosity. The Kanazawa equation evaluates viscosity ($\eta_{fl}$) of a purely viscous (Newtonian) fluid [4]. For Newtonian fluids the relation $\Delta\Gamma = -\Delta f$ applies. The Kanazawa equation is

$$\eta_{fl} = \frac{\Delta f^2}{\pi \rho_{fl} f_0 C_b^2} = \frac{\Delta\Gamma^2}{\pi \rho_{fl} f_0 C_b^2}$$

where $\rho_{fl}$ is fluid density and $C_b$ is the coefficient of proportionality, which depends on basic QCM characteristics under specific power supply conditions and temperature [5, 7]. The value for $C_b$ can be determined through calibration tests using standardised materials such as calibration oils of known viscosities and/or fixed-size solid plates. However, a limitation of the Kanazawa equation is that it is not generally applicable to study the mechanical characteristics of particles which couple to a resonator.

However, when the measurements in step 3 are taken the fluid is no longer Newtonian because particles within the fluid are interacting with (e.g. are coupled to) the resonator. In general, the mechanics of a single particle in the film in this situation can be described in terms of two-component mechanical system [5]. The first component is the part of the material bottom layer rigidly coupled to the resonator surface and second component is viscoelastic energy dissipative layer.

The rigidly coupled layer's mechanical characteristic is its mass density $M_p$, which depends on particle-to-surface interaction forces and particle shear modulus. The top layer's main characteristic is its complex shear modulus $G_p$, which consists of a real part $G_p'$ (called the storage modulus) and an imaginary part $G_p''$ (called the loss modulus).

Mechanical coupling or adhesion of viscoelastic material with progression of time causes a change of measured $\Delta f(t)$ and $\Delta \Gamma(t)$ values. These changes are dependent on three mechanical characteristics: the total mass density $M_{pl}(t)$ formed by bottom layer of rigidly coupled material, a total storage modulus $G_{pl}'(t)$ and a total loss modulus $G_{pl}''(t)$ formed by the top layer.

The ideal output result of any experiment to study viscoelastic property changes is the two sets of characteristics identified above, i.e. the three single particle properties ($M_p$, $G_p'$, $G_p''$) and the three time courses ($M_{pl}(t)$, $G_{pl}'(t)$, $G_{pl}''(t)$) of the properties of the film formed by all particles coupled at time t. However, this desired result is generally unachievable on a resonator because the output of a resonator gives only two measured parameters ($\Delta f$ and $\Delta \Gamma$). Thus, some theoretical simplification and/or reduction is required.

However, it is sufficient for a majority of such experiments to evaluate just two characteristics:

- a qualitative characteristic of a single particle, and
- a quantitative characteristic of total film formed by all particles coupled by the time t.

The qualitative and quantitative characteristics may be mapped directly to one of the properties identified above. For example, one mechanical characteristic of the set ($M_p$, $G_p'$, $G_p''$) may be extracted as the qualitative characteristic and one time course of the set ($M_{pl}(t)$, $G_{pl}'(t)$, $G_{pl}''(t)$) may be extracted as the quantitative characteristic. Alternatively, the qualitative and quantitative characteristics may comprise some other characteristics that may or may not be related to the properties identified above, i.e. some other qualitative characteristic being or not being a combination of ($M_p$, $G_p'$, $G_p''$) but having a clear physical meaning; and some other quantitative characteristic being or not being a combination of ($M_{pl}(t)$, $G_{pl}'(t)$, $G_{pl}''(t)$) but also having a clear physical meaning.

Previous attempts at extracting useful qualitative and quantitative characteristics have limited application and can give unphysical solutions if the assumptions used to derive them cease to apply.

For example, if particle size is relatively small or particle-to-surface interaction forces are strong enough to neglect the particle elastic properties, then one can assume that $G_p'=0$. The particles can be thus be considered as forming only a rigid layer. In this case, the Sauerbrey equation for evaluating mass density $M_{pl}$ of a film formed by coupled rigid particles can be applied [11]. It is possible to write in this case:

$$G_p''(t) = \frac{\Delta\Gamma(t)^2 - \Delta\Gamma(t_z)^2}{\pi\rho_{fl}f_0 C_b^2}$$

and

$$M_{pl}(t) = \frac{-(\Delta\Gamma(t) + \Delta f(t))}{2\pi f_0 C_b}$$

This approach is widely used in practice [5,7,13]. However, its applicability is limited to special cases where $G_p'$ is negligible. If the coupling particles are big or coupling to a surface is not firm then this assumption does not apply and characteristic $G_p'$ must be taken into account. In this case the above can lead to unrealistic negative mass density values known as the "missing mass effect" [14].

Moreover, these equations assume that coupling does not lead to an additional $\Delta\Gamma$ increase or if the additional $\Delta\Gamma$ increase is negligible [16]. Otherwise, i.e. if coupling does lead to significant additional $\Delta\Gamma$ increase, the calculated $M_{pl}(t)$ value will be always be an underestimated measure of the real mass density, reaching sometimes absurd negative values

In the particular case of investigating red blood cell (RBC) sedimentation in human blood in contact with a QCM (with gold electrodes) surface, it was assumed that cell-surface interaction forces are extremely weak [12]. Based on this assumption, the $M_{pl}$ value can be ignored, and the problem can be reduced to modified Borovikov equations [15] with an assumption that particle density is close to fluid density:

$$G'_{pl}(t) = \left(\frac{1}{\rho_{fl}C_b^2}\right)(\Delta\Gamma(t)^2 - \Delta f(t)^2)$$

and

$$G''_{pl}(t) = \left(\frac{1}{\rho_{fl}C_b^2}\right)(-2\Delta f(t)\Delta\Gamma(t) - 2\Delta\Gamma(t_z))$$

In this case, the approach is valid only for cases where $M_{pl}$ is negligible. If this assumption does not apply, the elasticity (i.e. storage modulus values $G'_{pl}(t)$) obtained using this approach can result in unrealistic negative values.

It is evident that the approaches described above are not universal. Moreover, it is only possible to evaluate some quantitative characteristics of the film formed by coupled particles. A single particle qualitative characteristic is not evaluated.

There are several recently published works which demonstrate the sensitivity of this method when applied to blood analysis [3, 12, 13,] using a QCM. Single fibre segments of the fibrin polymer have a length ranging from approximately 50 to 5000 nm [17, 18] and are considered by the majority of researchers to be purely elastic with typical $G'_p$ values of between 1 to 10 MPa [13, 17]. However, the whole fibrin clot as the polymer network is surrounded by viscous plasma induces both viscous and elastic responses on the sensor. The forces of interaction between fibres and the deposited coating material may cause the formation of rigid segments at points of contact. Hence, this implies that the same three mechanical characteristics can be studied.

This technique can also be applied to blood proteins. It has been demonstrated that plasma proteins (especially fibrinogen and albumin) are very absorbable to hydrophobic surfaces such as polystyrene [20, 21]. Plasma proteins have small sizes (1-50 nm) relative to the shear wave penetration depth. The typical values of their elasticity are approximately 1-10 GPa [22, 23]. The protein-to-surface interaction forces are strong, which means plasma proteins may be considered as absolutely rigid elements, characterized by just one mechanical parameter of mass density.

The journal article "Measurement of the evolution of rigid and viscoelastic mass contributions from fibrin network formation during plasma coagulation using quartz crystal microbalance" by Lakshmanan, et al. (Sensors and Actuators B, 192, p. 23-28) discloses study of the effect of fibrinogen concentration in the coagulation of blood plasma using a QCM.

US 4,862,384 discloses a method of measuring the dynamic viscosity by using an acoustic transducer.

The journal article "Investigation of prothrombin time in human whole-blood samples with a quartz crystal biosensor" by Müller, et al. (Anal. Chem. 2010, 82, 658-663) discloses a method of measuring coagulation time of human whole-blood samples.

US 2011/0129929 discloses an apparatus and method for monitoring a change of density and/or viscosity within a test sample.

The journal article "The oscillation frequency of a quartz resonator in contact with a liquid" by Kanazawa, et al. (Analytica Chimica Acta 175 (1985) 99-105) discloses a relationship that expresses the change in oscillation frequency of a quartz crystal in contact with a fluid. US 2013/0211748 discloses a method for measuring a viscoelastic modulus of a substance which forms a film on the surface of piezoelectric element, wherein at least two of a resonance frequency and half-value frequencies F1, F2 are used to calculate a mass load term and viscoelastic moduli of the film.

SUMMARY OF THE INVENTION

At its most general, the present invention provides an analysis technique that can determine complex qualitative and quantitative contributions to the viscoelastic properties of a film in contact with or in close proximity to a surface. The invention is based on an assumption that the viscosity of the surface of a fluid film that is opposite to a surface at which the film interacts with the surface changes negligibly, which in turn allows the derivation of new representative parameters for the mass and elasticity of the film as a whole as well as a qualitative parameter that is representative of a typical single particle within the film. These new representative parameters can be used to classify the film, and in the case of blood or other bodily fluids, can be used to identify pathological conditions. The invention is applicable to all types of fluid that physically interact with a surface, e.g. by exhibiting a coupling effect, adhesion or the like. Thus, the invention may be applied to industrial liquids as well as bodily fluids.

In one aspect, the invention provides a method of evaluating viscoelastic properties of a fluid film as it interacts with a surface, the method comprising: applying a fluid under test to an oscillating surface of a mechanical resonator sensor, wherein the fluid comprises particles, which, after the fluid under test is applied to the oscillating surface, begin to couple to the oscillating surface to form a film that interacts with the oscillating surface; detecting a change in resonant frequency $\Delta f$ and a change in half bandwidth $\Delta\Gamma$ of a resonance peak exhibited by the mechanical resonance sensor as the fluid under test forms the film that interacts with the oscillating surface; and evaluating two or more of: a first quantitative characteristic indicative of an effective mass $M_{\tilde{t}}$ of the film interacting with the oscillating surface, a second quantitative characteristic indicative of an effective elasticity $\mu_{\tilde{t}}$ of the film interacting with the oscillating surface, and a qualitative characteristic indicative of a rigidity factor $\varphi$ of an individual particle within the film interacting with the oscillating surface, wherein:

$$M_t^{\sim} = B\left[\Delta f(t_z) - \Delta f(t) - \frac{\Delta\Gamma(t_z)^2}{\Delta\Gamma(t)} + \Delta\Gamma(t_z)\right]$$

$$\mu_t^{\sim} = A\left[\frac{\Delta\Gamma(t)^2}{\Delta\Gamma(t_z)^2} - \frac{\Delta\Gamma(t_z)^2}{\Delta\Gamma(t)^2}\right]$$

and

$$\varphi = C\frac{\mu_t^{\sim}}{M_t^{\sim}}$$

where A, B and C are coefficients of proportionality, $\Delta f(t)$ and $\Delta\Gamma(t)$ are respectively the detected change in resonant frequency and half bandwidth of the resonant peak at a detection time $t$ when the film has formed and is interacting with the oscillating surface, and $\Delta f(t_z)$ and $\Delta\Gamma(t_z)$ are respectively the detected change in resonant frequency and half bandwidth of the resonant peak at a pre-interaction time $t_z$, which is after the fluid is applied but before any particles couple to the oscillating surface, where $t_z < t$.

The interaction between the film and the oscillating surface may be a physical interaction or coupling arising from the close proximity or contact of the fluid with the surface. For example, the fluid may form a film adhered to the surface. In this case, the pre-interaction time $t_z$ may be a pre-adhesion time, i.e. a point in time before adhesion occurs.

The method may evaluate all three model parameters. The evaluated model parameters may correspond exactly to the effective mass $M_{\tilde{t}}$, effective elasticity $\mu_{\tilde{t}}$ and rigidity factor $\varphi$, or they may be equivalent expressions which are based on the same underlying relationship between the measured changes in resonant frequency $\Delta f$ and half bandwidth $\Delta\Gamma$. For example, the expressions above are normalized with respective to the corresponding properties at the pre-interaction time $t_z$, but non-normalized expressions may also be used.

The method may include outputting a result of evaluating one or more of the first quantitative characteristic, the second quantitative characteristic and the qualitative characteristic. The outputting may comprise displaying information, e.g. in a graphical or numerical manner, that is indicative of the evaluated information.

The evaluating step may be performed repeatedly or continuously as coupling between the film and surface develops. The measured changes in resonant frequency $\Delta f$ and half bandwidth $\Delta\Gamma$ may vary over time. The output result may thus be time-series data for one or more of the first quantitative characteristic, the second quantitative characteristic and the qualitative characteristic. The method may include displaying the time-series data in a graphical manner, i.e. to show the evolution of the magnitude of the characteristic(s) with time.

The method may include comparing a result of evaluating one or more of the first quantitative characteristic, the second quantitative characteristic and the qualitative characteristic with a threshold value to classify the fluid under test. Comparing a result may include extracting a relevant parameter from one or more of the characteristics, e.g. an average or maximum rate of change or a maximum value, and comparing the value of the relevant parameter with the threshold value.

The changes in resonant frequency $\Delta f$ and half bandwidth $\Delta\Gamma$ of the resonance peak may be calculated relative to a resonant frequency $f_0$ and half bandwidth $\Gamma_0$ of the mechanical resonator sensor before the fluid is applied to the oscillating surface, i.e. resonant properties of the mechanical resonator sensor when unloaded or operating in air.

The fluid may behave as a Newtonian fluid at the pre-interaction time $t_z$, which means that $\Delta\Gamma(t_z) = -\Delta f(t_z)$, whereby the expressions for the characteristics can be simplified.

The method may include measuring values for the resonant frequency $\Delta f$ and half bandwidth $\Delta\Gamma$ directly from

properties of a local maximum in the conductance spectrum of a piezoelectric resonator, e.g. using an impedance analyser. However, detecting a change in half bandwidth $\Delta\Gamma$ may alternatively include determining a dissipation value $D$ or a Q-factor value $Q$, since these values are related to the half bandwidth $\Delta\Gamma$.

The fluid under test may be blood (e.g. whole blood) or a blood component (e.g. plasma) or other bodily fluid. The method may be able to extract information about properties of a blood component from a test performed on whole blood. For example, the method may include determining information indicative of any one or more of: blood clotting time, red blood cell deformability, platelet aggregability, erythrocyte sedimentation, thrombotic risk, and fibrinogen activity based on one or more of the first quantitative characteristic, the second quantitative characteristic and the qualitative characteristic.

The method may include identifying a pattern in one or more of the first quantitative characteristic, the second quantitative characteristic and the qualitative characteristic, and determining a pathological condition based on the identified pattern.

The method outlined above may be implemented on a computer using suitable executable instructions. In another aspect, the invention therefore comprises a computer program product comprising instructions stored on a computer-readable medium that are executable by a computer to perform the steps of: receiving input data comprising a change in resonant frequency $\Delta f$ and a change in half bandwidth $\Delta\Gamma$ of a resonance peak exhibited by a mechanical resonance sensor as a fluid under test forms a film interacting with an oscillating surface of the mechanical resonance sensor, wherein the fluid comprises particles, which, after the fluid under test is applied to the oscillating surface, begin to couple to the oscillating surface to form the film that interacts with the oscillating surface; evaluating two or more of: a first quantitative characteristic indicative of an effective mass $M_{\tilde{t}}$ of the film interacting with the oscillating surface, a second quantitative characteristic indicative of an effective elasticity $\mu_{\tilde{t}}$ of the film interacting with the oscillating surface, and a qualitative characteristic indicative of a rigidity factor $\varphi$ of an individual particle within the film interacting with the oscillating surface; and outputting the first quantitative characteristic, the second quantitative characteristic or the qualitative characteristic, wherein:

$$M_{t}^{\sim} = B\left[\Delta f(t_z) - \Delta f(t) - \frac{\Delta\Gamma(t_z)^2}{\Delta\Gamma(t)} + \Delta\Gamma(t_z)\right]$$

$$\mu_{t}^{\sim} = A\left[\frac{\Delta\Gamma(t)^2}{\Delta\Gamma(t_z)^2} - \frac{\Delta\Gamma(t_z)^2}{\Delta\Gamma(t)^2}\right]$$

and

$$\varphi = C\,\frac{\mu_{t}^{\sim}}{M_{t}^{\sim}}$$

where $A$, $B$ and $C$ are coefficients of proportionality, $\Delta f(t)$ and $\Delta\Gamma(t)$ are respectively the detected change in resonant frequency and half bandwidth of the resonant peak at a detection time t when the film has formed and is interacting with the oscillating surface, and $\Delta f(t_z)$ and $\Delta\Gamma(t_z)$ are respectively the detected change in resonant frequency and half bandwidth of the resonant peak at a pre-interaction time $t_z$, which is after the fluid is applied but before any particles couple to the oscillating surface, where $t_z < t$. As above, the interaction may include adhesion of the film on the surface.

The input data may be communicated from a remote location, e.g. over a computer network or the Internet. The outputted results may be communicated back to the same or another remote location. The invention may thus comprise one or more client terminals associated with the mechanical resonator sensor for obtaining (i.e. measuring or otherwise detecting) the input data, and a server in communication with the client terminal(s) over a computer network for receiving and processing the input data and generating and distributing output data corresponding to the results of the evaluation. The evaluating step may have any of the features of the method discussed above.

In another aspect, the invention provides an apparatus for evaluating viscoelastic properties of a fluid film that interacts with a surface, the apparatus comprising: a mechanical resonator sensor having an oscillatable surface for receiving a fluid under test, wherein the fluid comprises particles, which, after the fluid under test is applied to the oscillatable surface, begin to couple to the oscillatable surface to form a film that interacts with the oscillatable surface; and a processing device in communication with the mechanical resonator sensor to receive therefrom a change in resonant frequency $\Delta f$ and a change in half bandwidth $\Delta\Gamma$ of a resonance peak exhibited by the mechanical resonance sensor as the fluid under test forms the film interacting with the oscillatable surface during oscillation thereof, wherein the processing device is arranged to calculate output data representative of properties of the fluid under test, the output data including one or more of: a first quantitative characteristic indicative of an effective mass $M_{\tilde{t}}$ of the film interacting with the oscillating surface, a second quantitative characteristic indicative of an effective elasticity $\mu_{\tilde{t}}$ of the film interacting with to the oscillating surface, and a qualitative characteristic indicative of a rigidity factor $\varphi$ of an individual particle within the film interacting with the oscillating surface, wherein:

$$M_{\tilde{t}} = B\left[\Delta f(t_z) - \Delta f(t) - \frac{\Delta\Gamma(t_z)^2}{\Delta\Gamma(t)} + \Delta\Gamma(t_z)\right]$$

$$\mu_{\tilde{t}} = A\left[\frac{\Delta\Gamma(t)^2}{\Delta\Gamma(t_z)^2} - \frac{\Delta\Gamma(t_z)^2}{\Delta\Gamma(t)^2}\right]$$

and

$$\varphi = C\frac{\mu_{\tilde{t}}}{M_{\tilde{t}}}$$

where $A$, $B$ and $C$ are coefficients of proportionality, $\Delta f(t)$ and $\Delta\Gamma(t)$ are respectively the detected change in resonant frequency and half bandwidth of the resonant peak at a detection time $t$ when the film has formed and is interacting with the oscillating surface, and $\Delta f(t_z)$ and $\Delta\Gamma(t_z)$ are respectively the detected change in resonant frequency and half bandwidth of the resonant peak at a pre-interaction time $t_z$, which is after the fluid is applied but before any particles couple to the oscillating surface, where $t_z < t$. As above, the interaction between the film and the oscillating surface may be adhesion.

The processing device may be a conventional computer, e.g. programmed according to software instructions mentioned above. The mechanical resonator sensor may comprise a piezoelectric resonator, which may be excited by an AC source. An impedance analyser may be used to measure the resonant properties from the conductance spectrum of the piezoelectric resonator. In one implementation the mechanical resonator sensor comprises a quartz crystal microbalance, but other devices may be used, e.g. any type of Thickness Shear Mode (TSM) resonator or Face Shear Mode (FSM) resonator.

At a diagnostic level, the invention may be applied to allow all the individual contributions to the viscoelastic behaviour of blood to be determined. From this complex information the overall viscoelastic status of the blood may be determined, which in turn can assist in the identification of the source of any dysfunction. Thus, it allows clinicians to determine both the cause and effect of a pathological condition such as venous thromboembolism (VTE) in that it allows the identification of the underlying cause (e.g. elevated fibrinogen levels), while also showing the potential threat that the condition causes, e.g. in terms of a global index of risk determined by the behaviour of the blood sample in its entirety.

The global index of risk may take into account other factors that relate to blood viscoelastic properties, which may be measured at the same time, either within the same test, or with a number of suitably selected parallel tests.

In addition, the invention may enable identification of new patterns of viscoelastic behaviour not linked to known causes of venous thromboembolic risk, which may allow the identification of individuals who have presented with a VTE, but have no identifiable risk factors as determined by current test methodologies. Thus, the method of the invention may be applied to one or more of blood samples from patients having a known pathological condition, and the method may include determining a correlation between the evaluated viscoelastic properties of the blood during coagulation (i.e. adhesion) on the oscillating surface. The correlations may be determined using conventional techniques. A correlation may be determined for a plurality of pathological conditions and may be stored e.g. in a computer memory to permit comparison with a blood sample from a patient with an unknown condition to facilitate identification (i.e. diagnosis) of that condition.

BRIEF DESCRIPTION OF THE DRAWINGS

A derivation of the principles underlying the invention and a number of practical examples are explained below in detail with reference to the accompanying drawings, in which:

Fig. 1 shows a simple mechanical model describing blood behaviour on a resonator;
Fig. 2 is a graph showing a theoretical data representation on the complex frequency plane {-Δf,ΔΓ};
Fig. 3 is a graph showing changes over time of QCM spectral characteristics (-Δf,ΔΓ) for whole blood (WB) and platelet poor plasma (PPP);
Fig. 4 is a graph showing changes over time of QCM spectral characteristics (-Δf,ΔΓ) for whole blood (WB) and platelet rich plasma (PPP);

Fig. 5 is a graph showing a time-course analysis of QCM spectral characteristics ($-\Delta f, \Delta \Gamma$) for PRP at times after the z-point;

Fig. 6 is a graph showing a time-course analysis of QCM spectral characteristics ($-\Delta f, \Delta \Gamma$) for WB at times after the z-point;

Fig. 7 is a graph showing changes over time of QCM spectral characteristics ($-\Delta f, \Delta \Gamma$) coagulation under APTT-type test conditions for PPP with and without extra fibrinogen concentration;

Fig. 8 is a graph showing a time-course analysis for the QCM spectral characteristics shown in Fig. 7;

Fig. 9 is a graph showing a time-course analysis of QCM spectral characteristics ($-\Delta f, \Delta \Gamma$) for PPP at times after the sample activation using three different concentrations of tissue factor;

Fig. 10 is a graph showing the time-course analysis of rigidity factor corresponding to QCM spectral characteristics from Fig. 9, plotted for the three tissue factor concentrations;

Fig. 11 shows scanning electron microscope (SEM) images of the clot formed on the resonator surface corresponding to three concentrations of TF; and

Fig. 12 shows a pair of graphs which illustrate changes in rigidity factor for normal and glutaraldehyde-treated red blood cells (RBC).

## DETAILED DESCRIPTION; FURTHER OPTIONS AND PREFERENCES

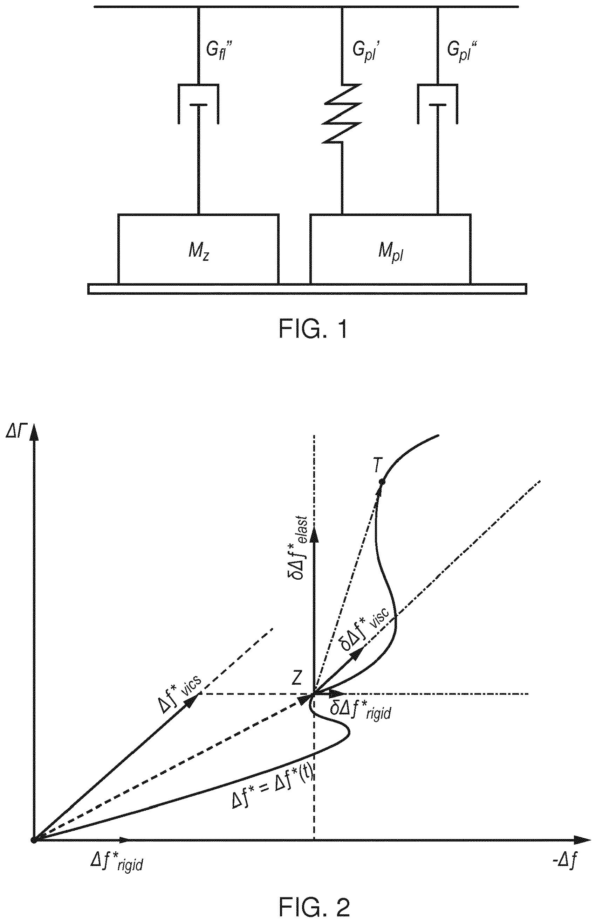

Fig. 1 shows a simple circuit model that can be used to study the process of particle adhesion to a surface from viscous fluid. It is assumed that the sensor response consists of three phases during adhesion experiment, corresponding to:

1. operation in unloaded state (i.e. where the sensor operates in air);
2. operation after immersion in fluid but before onset of adhesion;
3. operation after onset of particle adhesion to the sensor surface.

At phase 2 a time point $t_z$ is defined as the "z-point". The sensor response here is characterized by two elements: fluid viscosity $\eta_{fl} = G_{fl}''/2\pi f_0$ and parasitic rigid mass density $M_z$ whose values can be easily determined.

In phase 3, the response is characterized by three elements, namely storage modulus $G_{pl}'(t)$, loss modulus $G_{pl}''(t)$, and rigid mass density $M_{pl}(t)$ of a layer formed by particles coupling to the sensor surface at $t > t_z$.

At the z-point, the fluid is typically ideally viscous, i.e. the relation $\Delta\Gamma(t_z) = -\Delta f(t_z)$ applies and the Kanazawa equation can be used to define fluid viscosity. However, in some experiments a parasitic mass is observed as $\Delta\Gamma(t_z) < -\Delta f(t_z)$. The parasitic mass may be caused by surface roughness of the sensor [7] and/or some fast absorption of small rigid particles immediately after fluid loading. In any case it is possible to define both mechanical characteristics of the z-phase [5,7]:

$$G_{fl}'' = 2\pi f_o \eta_{fl} = \frac{2\Delta\Gamma(t_z)^2}{\rho_{fl} C_b^2}$$

..Equation 1

and

$$M_z = \frac{-(\Delta\Gamma(t_z) + \Delta f(t_z))}{2\pi f_0 C_b}$$

..Equation 2

where $\rho_{fl}$ is fluid density, and $C_b$ is the coefficient of proportionality dependent on QCM basic characteristics under specific power supply conditions and temperature [6,7]. As mentioned above, the value of $C_b$ can be determined through calibration tests using standardised materials such as calibration oils of known viscosities and/or fixed-size solid plates [7]. However, if $\Delta\Gamma(t_z) = -\Delta f(t_z)$ there is no need to take a parasitic mass into account.

After the z-point when adhesion begins we have to evaluate three unknown parameters. The general expression for complex frequency shift according to circuit model shown in Fig. 1 can be written in the following form [7] for a particle density of $\rho_p$:

$$\Delta f(t) + j\Delta\Gamma(t) = jC_b\sqrt{\left(jG_{fl}''\rho_{fl} + jG_{pl}''(t)\rho_p + G_{pl}'(t)\rho_p\right)} - C_b 2\pi f_0\big(M_{pl}(t) + M_z\big)$$

..Equation 9

This equation is unsolvable. The number of variables needs to be reduced. Nevertheless, some preliminary analysis can be done by plotting data points {-$\Delta f(t)$,$\Delta\Gamma(t)$} on the complex frequency plane, as shown in Fig. 2. In this regard, the sensor response is characterised by the trace of vector-function $\Delta f^*(t)$=-$\Delta f(t)$\{**1,0**\}+$\Delta\Gamma$\{**0,1**\}. In principle, it is possible to perform $\Delta f^*(t)$ decomposition not only in an orthogonal basis but also into vector components concerned with the exact mechanical properties of a substance. For instance, at the z-point, a vector $\Delta f_z^*$ can be represented as the sum of a "viscous vector" $\Delta f_{visc}^*$ and a "rigid vector" where $\Delta f_{rigid}^*$,

$$\Delta f_{visc}^* = C_b\sqrt{\frac{\pi\rho_{fl}f_0\eta_{fl}}{2}}\{\mathbf{1,1}\}$$

and

$$\Delta f_{rigid}^* = C_b 2\pi f_0 M_z\{\mathbf{1,0}\}$$

After the z-point, i.e. when extra viscosity, rigidity and elasticity emerge, the following corresponding vector additives come into play:

$$\Delta f^*(t) = \Delta f_{visc}^* + \Delta f_{rigid}^* + \delta\Delta f_{visc}^*(t) + \delta\Delta f_{rigid}^*(t) + \delta\Delta f_{elast}^*(t)$$

It follows that if viscosity and elasticity do not vary, then any changes in rigid mass lead to data point shifts only in the horizontal direction (i.e. -$\Delta f$). In contrast, elastic changes at stable viscosity and rigid mass displace data points vertically, i.e. parallel to the $\Delta\Gamma$ axis. Purely viscous metamorphoses occur at an angle of 45°, i.e. in the direction corresponding to equal changes of $\Delta\Gamma$ and -$\Delta f$. After the z-point, there is no unambiguous solution, and the exact position of a given point T can be due to an infinite number of possible combinations of vector additives.

The present invention is based around a manipulation of the equation above in which the viscous additive $\delta\Delta f_{visc}^*(t)$ is always treated as a linear sum of $\delta\Delta f_{rigid}^*(t)$ and $\delta\Delta f_{elast}^*(t)$. This permits this term to be excluded from the modelling approach. In effect one passes to a new set of three additive vectors:

$$\delta\Delta f_{eff.visc}^*(t) = \mathbf{0}$$

$$\delta\Delta f_{eff.rigid}^*(t) = \delta\Delta f_{rigid}^*(t) + \frac{1}{\sqrt{2}}\left(\delta\Delta f_{visc}^*(t)\{\mathbf{1,0}\}\right)$$

$$\delta\Delta f_{eff.elast}^*(t) = \delta\Delta f_{elast}^*(t) + \frac{1}{\sqrt{2}}\left(\delta\Delta f_{visc}^*(t)\{\mathbf{0,1}\}\right)$$

which provide an unambiguous solution in terms of "effective rigid mass density" and "effective elasticity". Of course, there are two other equivalent ways to reduce number of unknown variables: represent $\delta\Delta f_{rigid}^*(t)$ as linear sum of $\delta\Delta f_{elast}^*(t)$ and $\delta\Delta f_{visc}^*(t)$ or represent $\delta\Delta f_{elast}^*(t)$ as linear sum of $\delta\Delta f_{visc}^*(t)$ and $\delta\Delta f_{rigid}^*(t)$. However, this may lead to limitations in a similar manner to the known approaches discussed above. A clear advantage of the invention is that the resulting "effective" values will not have paradoxical negative values. Where $G_{pl}''(t) \equiv 0$ and

after substitution of values $\eta_{fl}$ and $M_z$, the following relations can be obtained from Equation 9:

$$G'_{pl}(t) = \frac{2}{\rho_p C_b^2}\left(\Delta\Gamma(t)^2 - \frac{\Delta\Gamma(t_z)^4}{\Delta\Gamma(t)^2}\right)$$

..Equation 3

and

$$M_{pl}(t) = \frac{1}{2\pi f_0 C_b}\left(-\Delta f(t) - \frac{\Delta\Gamma(t_z)^2}{\Delta\Gamma(t)}\right) - M_z$$

..Equation 4

$M_{pl}(t)$ and $G'_{pl}(t)$ calculated using the equations above can thus be considered as universal for both absolutely rigid and partially rigid particles. Indeed, the equation for $M_{pl}(t)$ in the case of absolutely rigid particle adhesion coincides with the Sauerbrey equation for $M_{pl}(t)$ due to the fact that $\Delta\Gamma(t) = \Delta\Gamma(t_z)$ as a criterion of rigidity.

As mentioned above, the term "effective" is used to characterise $M_{pl}(t)$ and $G'_{pl}(t)$ because their values both contain information about the viscosity of the film formed by coupled particle top layer. In other words, the information about viscosity is distributed between $M_{pl}(t)$ and $G'_{pl}(t)$ values. The present invention suggests using the calculated $M_{pl}(t)$ and $G'_{pl}(t)$ values as the quantitative characteristics of the film formed by all particles coupled to the sensor at time t.

In addition, the invention suggests using the ratio

$$F_p = G'_{pl}/M_{pl}$$

..Equation 5

as a qualitative characteristic of a single coupled particle after its adhesion to a particular type of surface. The SI unit for $F_p$ is [N/kg] and the value of $F_p$ depends on many factors including conditions of exact experiment. In general, however, the value of $F_p$ can be used as a qualitative indicator of contributions due to elastic and rigid components of coupled particle. For the case where rigid mass contribution is significantly larger than contribution of elasticity, i.e. $M_{pl} \gg G'_{pl}$, $F_p$ tends to zero. Alternatively, for a case where $M_{pl} \ll G'_{pl}$ and $F_p$ tends to infinity, indicating that elasticity component is more dominant.

The effective elasticity $G'_{pl}$ and the effective mass $M_{pl}$ density may be normalized to yield a normalized effective elasticity $\mu_{\tilde{t}}$ and normalized effective mass $M_{\tilde{t}}$ as follows:

$$\mu_{\tilde{t}} = \frac{G'_{pl}(t)}{G''_{fl}(t_z)} = A\left[\frac{\Delta\Gamma(t)^2}{\Delta\Gamma(t_z)^2} - \frac{\Delta\Gamma(t_z)^2}{\Delta\Gamma(t)^2}\right]$$

..Equation 6

and

$$M_{\tilde{t}} = B\left[\Delta f(t_z) - \Delta f(t) - \frac{\Delta\Gamma(t_z)^2}{\Delta\Gamma(t)} + \Delta\Gamma(t_z)\right]$$

..Equation 7

where $A$ and $B$ are coefficients of proportionality. Although normalisation is not necessary, it allows the expression of $\mu_{\tilde{t}}$ to exclude an unknown $\rho_p$ parameter and also permits relative changes of viscoelastic parameters with respect to fluid loss modulus value to studied. For $M_{\tilde{t}}$ it takes into account mass changes occurring beyond the z-point.

An effective rigidity factor $\varphi$ corresponding to the normalized mechanical characteristic of a viscoelastic particle when it is coupled to a particular surface can be calculated as

$$\varphi = C \frac{\widetilde{\mu_t}}{\widetilde{M_t}}$$

..Equation 8

where $C$ is a coefficient of proportionality.

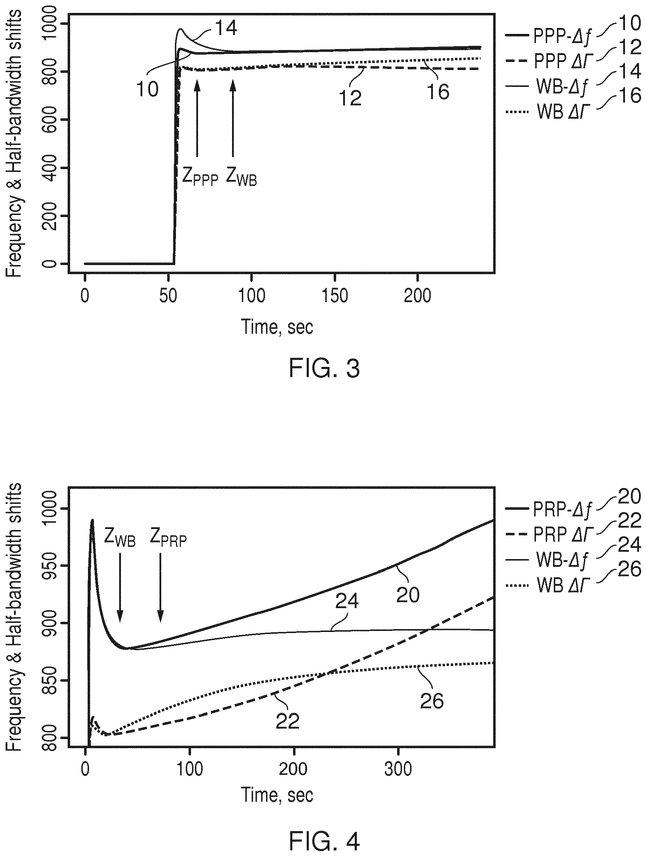

Various examples of how the method of the present invention may be put into practice are discussed below with reference to Figs. 3 to 9. The resonator used in the examples is a well-established quartz crystal microbalance (QCM). For QCM based measurements, the shear oscillation wave penetration depth in viscous medium can be estimated as [12]:

$$\delta = \frac{\eta}{\pi \rho f_0}$$

Plasma usually demonstrates almost purely Newtonian behaviour in a wide range of frequencies [12, 19] up to at least 100 MHz. Normal plasma viscosity $\eta$ = 1.2 mPas and if $f_0$ = 5 MHz gives $\delta$ = 273 nm. Thus, the plasma volume undergoing shear perturbations is much smaller than, for example, the typical size of a red blood cell. Oscillations occur close to the surface and are unable to perturb blood cells until they are attached. This is the specificity of the QCM method in contrast to low-frequency rheometry where the plasma movement occurs at distances $\delta$ > 100um, and this process incorporates single RBCs and RBC ensembles in the oscillation process. Indeed, erythrocytes are considered to be the primary factor for blood non-viscosity under 100 Hz [19]. In contrast, for QCM - which operates with a frequency of 5 MHz - there is no difference between plasma and whole blood (WB), which suggest almost Newtonian-like behaviour.

On the other hand, when a blood cell couples to the QCM surface, shear waves start propagating through it. RBC are typically considered to be "soft" objects. More specifically, the elasticity of a single RBC (concerned mainly with its membrane characteristics) falls in the range of between 1 and 40 kPa, depending on the method of analysis selected for use [24], and the inner cell viscosity ranges between approximately 2-10 mPas [25]. Thus, after coupling between the erythrocyte and the sensor surface, waves are propagating inside the cell have a decay length shorter than 1 $\mu$m and are not able to reach the top of the cell. Therefore, QCM can discern only a single layer of cells occupying the sensor surface.

There are three parameters of blood cells to examine: real and imaginary parts of the complex shear modulus $G'_p$ and $jG''_p$, characterising the cell's inner structure; and mass density of the rigid cell base $M_p$, which depend on both QCM coating type and the elastic properties of the cell. This consideration seems reasonable for any type of blood cell (RBCs, PLTs, etc.), as well as for cell clusters and aggregates formed upon adhesion.

Other types of blood components also attach to the surface and change the symmetric viscous sensors response appear upon the onset of coagulation. The blood clot and its main component, the fibrin polymer, is often studied by QCM.

Although the illustrated examples use a QCM resonator, the analysis method of the invention can be used with any shear mode resonator, and is applicable to both Thickness Shear Mode (TSM) and Face Shear Mode (FSM) resonators. For example, the invention may be used with any of the devices listed in Table 1.

Table 1: List of possible shear mode resonators

| Resonator | Mode | Frequency (air) | Q-value (air) | Q-value (liquid/water) |
|---|---|---|---|---|
| FBAR-Shear mode (ZnO) | TSM | 830 MHz<br>790 MHz | 380<br>285 | 199<br>100-150 |
| FBAR Shear mode (AlN) | TSM | 1.2-1.6 GHz | 350 | 150 |
| EMAT | TSM | 2-35 MHz | 7800-72000 | - |
| EMAT | FSM | 2-200 kHz | 3000 | 1419 |
| QWTSR | TSM | 730 MHz | 4400 | - |

In Table 1 FBAR is Film Bulk Acoustic Resonator, EMAT is Electro-Magnetic Acoustic Transducer, and QWTSR is Quarter-Wave-Thickness Shear Resonator.

Fig. 3 shows the response of the QCM resonator to platelet poor plasma (PPP) and whole blood (WB) loading. PPP and WB samples obtained from the same volunteer were used to study $-\Delta f$ and $\Delta\Gamma$ curves over time. In Fig. 3, the WB and PPP curves are aligned with respect to the time of sample loading. Sample loading occurs at $t = 54$ seconds. The initial response is a short (between 13 to 30 seconds) perturbation whilst the sensor passes non-monotonically to its new regime. Unfortunately, the pipette loading of a liquid substance on QCM operating in air is quite an abrupt procedure for the micromechanical sensor, so curve perturbations are routinely observed. The point in time domain where perturbations cease is marked as a pair of z-points $Z_{PPP}$ and $Z_{WB}$ on the graphs, and is used as a "start level" for further analysis. The z-point times are defined as follows: $t_z = 10$sec for PPP and $t_z = 28$sec for WB (with respect to the time of loading). It is easy to see that PPP and WB spectral characteristics are very similar at their z-points ($\Delta f \approx 810$ Hz and $\Delta \Gamma \approx 870$ Hz). This confirms the hypothesis that there is no distinction between blood and plasma behaviour on QCM before blood cell adhesion starts.

Furthermore, the observation that $-\Delta f > \Delta \Gamma$ at the z-points indicates the presence of some non-viscous factors which are not observed in calibration tests. One can propose that there is a rigid mass layer on the sensor surface formed by absorbed proteins (PPP and WB with the same protein concentration). Indeed, it has been demonstrated [21, 26] that for highly hydrophobic surfaces, plasma protein absorption occurs very rapidly, reaching 90% of its plateau value (approximately 1 $\mu$g/cm$^2$ of mass density) 4 to 8 seconds after loading. It is impossible to derive the kinetics of such a rapid process as shown in Fig. 3, simply because it is "hidden" by the perturbation of the sensor. At the z-point, it is only possible to observe the result of the protein absorption as the clear difference between $-\Delta f_z$ and $\Delta \Gamma_z$. In this case, Equations 1 and 2 above are applicable to define plasma viscosity and protein layer rigid mass densities. In more than 10 tests with normal blood and plasma the following values were achieved: $\eta_{fl} = 0.92 \pm 0.06$ mPas and $M_z = 1.0 \pm 0.5$ $\mu$g/cm$^2$. The diluted (1:1) plasma viscosity $\eta_{fl}$ can be recalculated to the undiluted one $\eta_{fl}^* = 1.16 \pm 0.12$ mPas, which corresponds to the normal range of values at 37°C [27]. The $M_z$ value exhibits a large deviation and can be used just as a qualitative diagnostic index. After the z-point, the PPP time responses 10, 12 remains substantially constant, which indicates that all processes are completed (i.e. there are no additional components left which can attach to the sensor surface). At the same, the time curve 16 for WB $\Delta \Gamma$ demonstrates a slow monotonous increase indicating the appearance of viscoelastic material on the sensor surface.

A first group of experiments was performed using platelet rich plasma (PRP) and whole blood (WB) obtained from the same healthy donor. Figs. 4 to 6 show the typical QCM response and corresponding model parameters analysis (i.e. $M_{\tilde{i}}(t)$, $\mu_{\tilde{i}}(t)$, $\varphi(t)$) with respect to platelet and RBC sedimentation. Similarly to Fig. 3, the $\Delta f$ and $\Delta \Gamma$ responses were aligned with respect to the time of sample loading. Consequently, z-points were then determined as $t_z = 26$ seconds for WB and $t_z = 30$ seconds for PRP. At the z-point, the QCM can probe viscosity of plasma and the rigid layer of absorbed plasma proteins only. The responses obtained for both look very similar, and Equations 1 and 2 can be applied to determine the following parameters: $\eta_{fl} = 0.92$ mPas and $M_z = 0.93$ $\mu$g/cm$^2$. After the z-points, at $t > t_z$ the responses differ significantly.

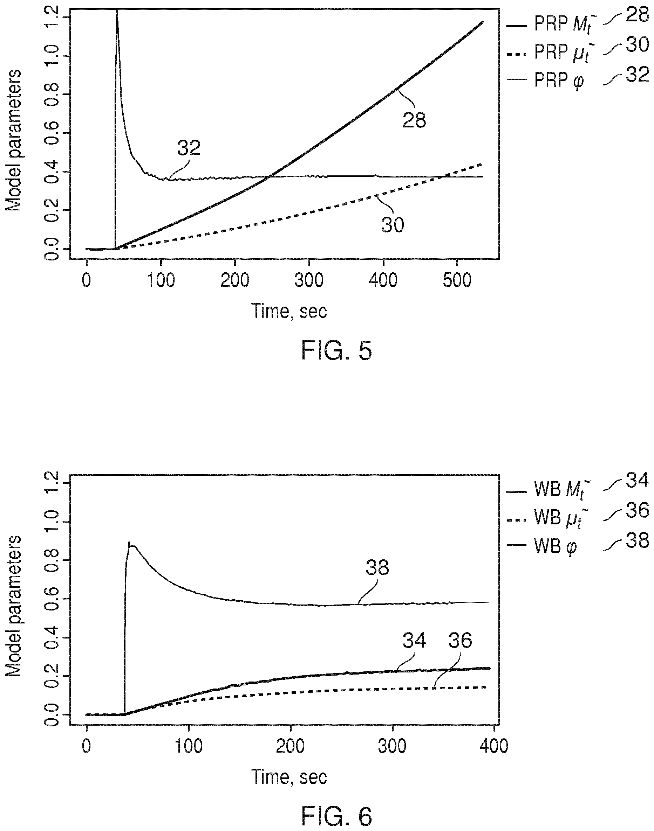

The primary component of PRP which plays a key role in the adhesion process is platelets. Equations 3 and 4 can give an interpretable and understandable picture of the platelet aggregation process better than just raw $-\Delta f$ and $\Delta \Gamma$ curves. In this case the analysis the $-\Delta f$ and $\Delta \Gamma$ curves is in terms of normalized effective elasticity $\mu_{\tilde{i}}$, normalized effective mass $M_{\tilde{i}}$ and rigidity factor $\varphi$ discussed above with reference to Equations 6 to 8. Fig. 5 shows the evolution of the time course of this properties for PRP and Fig. 6 shows the evolution of the time course of this properties for WB. These curves can be used to extract useful physical information as described below.

Firstly, the form of the PRP $M_{\tilde{i}}(t)$ curve 28 can be used for numerical estimation of aggregate mass growth rate (as a first derivative) measured in s$^{-1}$ (or in mg·cm$^2$sec$^{-1}$ by using an appropriate constant) and, if necessary, for second derivative analysis. Secondly, the PRP rigidity factor $\varphi(t)$ curve 32 has a form very close to "step-function". The platelet aggregates consist of a number of identical single fragments coupled to the sensor surface. Each fragment has the same pair of mechanical characteristics $M_{\tilde{i}}$ and $\mu_{\tilde{i}}$. When the number of coupled fragments increases, the total responses $M_{\tilde{i}}(t)$ and $\mu_{\tilde{i}}(t)$ proportional to that number will also increase, while, $\varphi(t)$ the rational parameter, being a characteristic of a single fragment does not change, indicating the advantage that the current invention has in terms of qualitatively identifying property of single fragments on the resonator surface. Thus, the rigidity factor analysis as two parameters monitoring (qualitative $\varphi$ and quantitative $M_{\tilde{i}}$) have a potential for study of individual features of platelet aggregation process.

In contrast to PRP, WB contains platelets and RBCs, and this has a significant impact on the QCM response, as can be seen by the differences between Figs. 5 and 6. The null hypothesis $M_{\tilde{i}}(t)$ curve 34 can be interpreted as being representative of RBC sedimentation dynamics. It is notable that mass density reaches saturation approximately 300 sec after the z-point, and the sedimentation rate (as reciprocal to the time of half-saturation) can be easily extracted from this graph. In comparison to other methods, one should take into account the fact that the QCM senses just a single layer of erythrocytes attached to the surface. The form of the rigidity factor curve for WB is not so clearly "step-shaped" which is indicative of the quite complicated multicomponent nature of WB. Nevertheless, its final value of about 0.6 indicates weaker cell-to-surface interactions and/or the softer structure of single RBC compared to platelet aggregates.

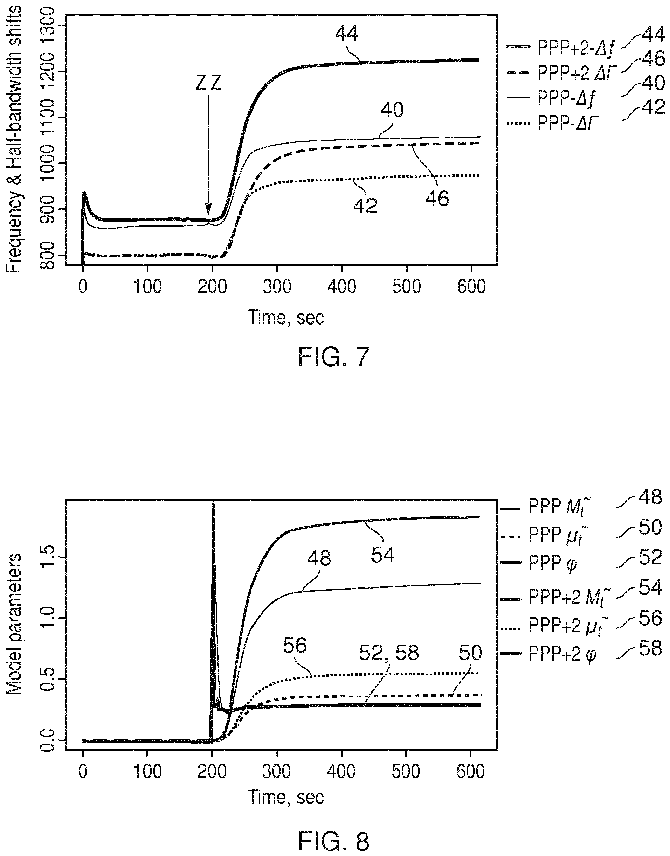

A second group of experiments was performed for plasma coagulation monitoring using platelet poor plasma (PPP) in the absence and presence of spiked fibrinogen to permit the study the dynamics of the plasma coagulation using the aPTT activation. Fig. 7 shows QCM responses in the form of $-\Delta f$ and $\Delta\Gamma$ curves 40, 42 for a sample in the absence of spiked fibrinogen (PPP) and $-\Delta f$ and $\Delta\Gamma$ curves 44, 46 for a sample having 2g/L of fibrinogen (PPP+2). The $-\Delta f$ and $\Delta\Gamma$ curves were aligned with respect to the time of sample loading. CaCl$_2$ solution was added to initiate fibrin polymerisation after approximately 190-200 seconds and the z-points were chosen as 5 seconds thereafter.

Fig. 8 shows the corresponding model parameters analysis, with $M_{\tilde{i}}(t)$, $\mu_{\tilde{i}}(t)$, $\varphi(t)$ curves 48, 50, 52 plotted for the PPP sample and $M_{\tilde{i}}(t)$, $\mu_{\tilde{i}}(t)$, $\varphi(t)$ curves 54, 56, 58 plotted for the PPP+2 sample.

It can be seen from Fig. 7 that the PPP and PPP+2 samples produce different responses at the z-point. This is a result of the different protein concentrations in the two samples. Using Equations 1 and 2, we may calculate $\eta_{fl}$ = 0.91 mPas for both samples, although $M_z$ = 0.94 $\mu$g/cm$^2$ for PPP and $M_z$ = 1.03 $\mu$g/cm$^2$ for PPP+2. After the z-points, at $t>t_z$, a lag-phase of 20-25 seconds was observed, which was followed by a dramatic increase in both QCM spectral characteristics corresponding to a fibrin network formation. One can observe differences in the changes in $-\Delta f$ and $\Delta\Gamma$ for PPP and PPP+2 samples resulting in larger magnitude of changes for a larger fibrinogen concentration.

However, interestingly, the results of rigidity factor $\varphi$ analysis for PPP and PPP+2 samples shown in Fig.8 exhibit a step-shaped form similar to the case of platelet aggregation.

The following information can be derived from Fig. 8:

i. $\varphi(t)$ is constant in time and is approximately 0.27.

ii. One can assume that the clot in the contact zone consists of identical single fibrin segments coupled to the QCM sensor surface. Each segment has the same pair of mechanical characteristics $M_{\tilde{i}}$ and $\mu_{\tilde{i}}$. In this instance, values of $M_{\tilde{i}}(t)$ and $\mu_{\tilde{i}}(t)$ are proportional to the increasing number of segments coupled at time $t$. Meanwhile, $\varphi$ is the rational qualitative parameter, being an attribute of single fibrin segment and hence, it does not change;

iii. The constant value of 0.27 is the same for the PPP and PPP+2 samples. Furthermore, it was detected that it was the same in more than 30 tests with normal plasma having wide range of fibrinogen concentrations. Hence, $\varphi$ can be considered as an important mechanical qualitative characteristic of normal fibrin polymer attached to a particular type of surface. One can expect that $\varphi$ is sensitive to the type of coagulation activation (KCT, PT, APPT, Tissue factor) and to the choice of QCM coating material (see $-\Delta f(t)$ and $\Delta\Gamma(t)$ curves presented in some publications [20, 28]).

iv. Relative rigidity and relative elasticity, $M_{\tilde{i}}(t)$ and $\mu_{\tilde{i}}(t)$, are linearly dependent through rigidity factor ($\varphi$). Therefore it is sufficient to use just one of them to study quantitative characteristics of clot formation in APTT-type tests. Relative rigidity $M_{\tilde{i}}(t)$ is probably useful as it is likely to be less noise-dependent (it contains just the first power of $\Delta f$ and $\Delta\Gamma$).

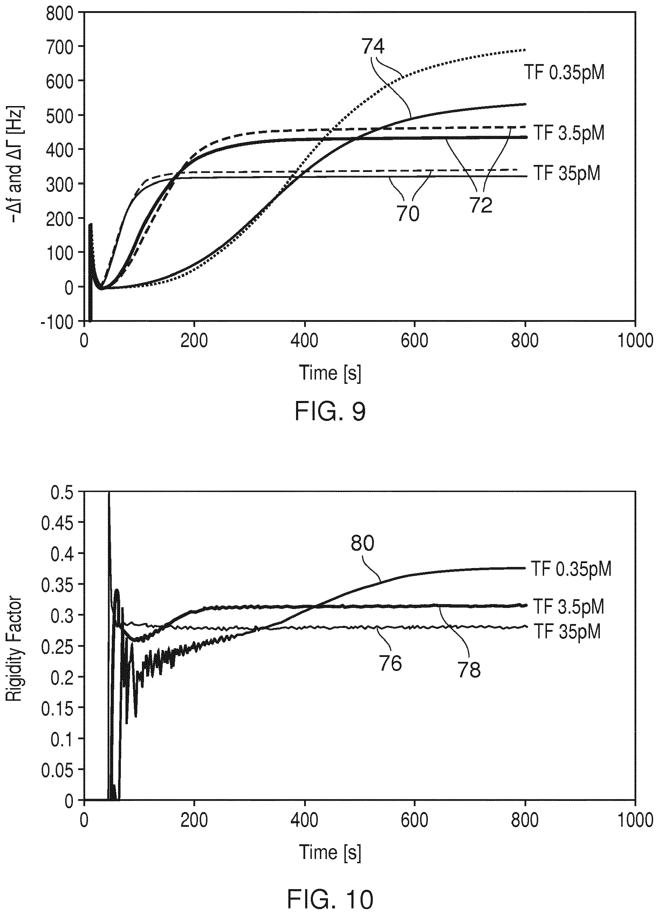

A third group of plasma coagulation monitoring experiments were performed using normal platelet poor plasma (PPP) and activated using different tissue factor concentrations. Fig. 9 shows QCM responses in the form of $-\Delta f$ (solid lines) and $\Delta\Gamma$ (dashed lines) and aligned to the time of addition of activation reagents. Fig. 9 shows curves 70, 72, 74 for the samples activated with 35, 3.5 and 0.35 pM concentration of TF, respectively. In this group the activators are mixed prior (10s) to the addition of the sample to the resonator and thus the z-point was chosen immediately after the addition of the sample to the resonator.

Fig. 9 shows that after the z-point, $t > t_z$ the lag phase was longer for smaller concentrations of TF followed by the characteristic dramatic increase in both $-\Delta f$ and $\Delta\Gamma$ corresponding to the fibrin network formation. The final steady state values for $-\Delta f$ and $\Delta\Gamma$ and the rate of clot formation was also dependent on the concentration of the activator. The rate of clot formation is associated with the rate of thrombin generated and thus would be expected to cause differences in the quality of the fibrin network formed.

Fig. 10 shows the corresponding rigidity factor analysis application, plotted for different tissue factor concentrations and shown in three curves 76, 78, 80 for concentrations of 35 pM, 3.5 pM and 0.35pM respectively. One can observe that the final rigidity factor characterising the formed clot was larger for samples activated with a smaller concentration of tissue factor. This result suggests that the rate of formation of clot observed in Fig. 9 causes differences in the quality of the clot.

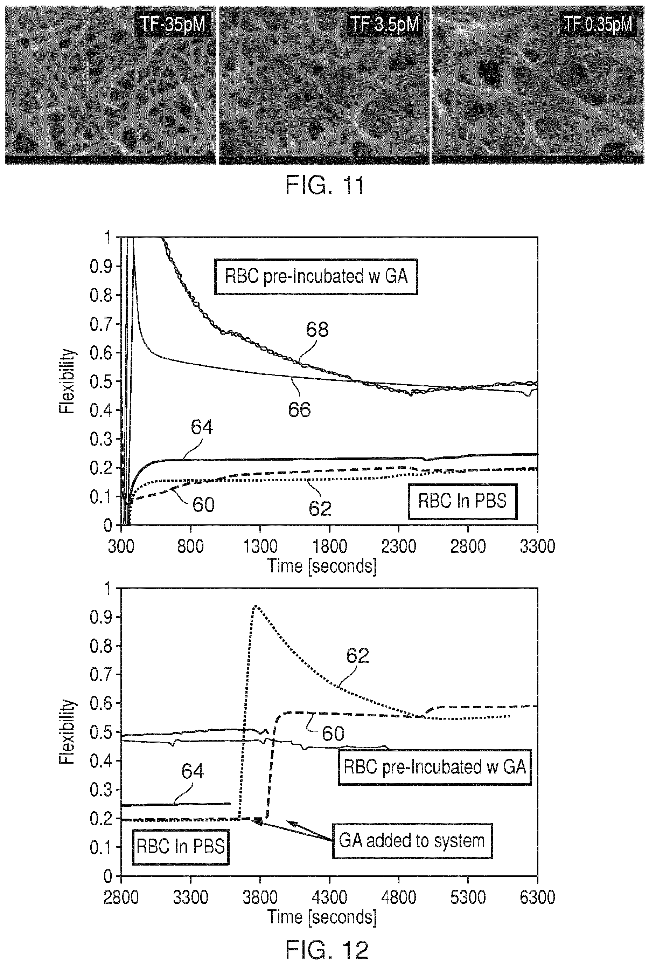

The SEM images obtained, shown in Fig. 11, show that a larger average fibrin diameter and lower fibrin network density was observed for a smaller concentration of TF. The proposed model used to evaluate rigidity factor predicted the differences in the nature quality of the formed clot resulting from different rates of thrombin generation for the three concentrations of tissue factor. This further supports the applicability of the proposed model to identify differences that current methods such as thromboelastography are unable to predict through their measure of clot strength.

Whilst the first set of experiments considered sedimentation of RBC from whole blood in a static system, a fourth set of experiments used the invention to evaluation a mechanical property of individual red blood cells coupled to the QCM.

The rigidity factor $\varphi$ is a parameter that was shown in the previous examples as a qualitative property of individual component that couples to the surface. The coupling of RBC to the surface can be achieved by electrostatic (poly-L-lysine) or covalent (immobilized antibody) coupling to the resonator surface. Under aqueous conditions, PLL confers a positively-charged hydrophilic amino group, which functions as a ligand for negatively-charged entities. In this example, a poly-L-lysine coated resonator was used and RBC suspensions were allowed to flow over the resonator. As the RBC electrostatically couples to the surface, it produces characteristic $\Delta f$ and $\Delta\Gamma$ responses. These responses can then be evaluated in terms of the effective mass and elasticity $M_{\tilde{i}}(t)$ and $\mu_{\tilde{i}}(t)$ using the proposed model.

The left hand graph in Fig. 12 shows how the introduction of RBC into the system results in a change in rigidity factor $\varphi(t)$ as the RBC comes in contact with the poly-L-lysine surface for three different sample. Curve 60 corresponds to a first sample with 1% RBC, curve 62 corresponds to a second sample with 0.1% RBC and curve 64 corresponds to a third sample with 0.01% RBC.

The experiment was repeated for an additional second sample (0.1% RBC) and an additional third sample (0.01% RBC) which had been pre-incubated with glutaraldehyde (GA), which reduces the deformability of RBC. Curve 66 is the rigidity factor $\varphi(t)$ for the pre-treated second sample (0.1% RBC). Curve 68 is the rigidity factor $\varphi(t)$ for the pre-treated third sample (0.01% RBC). The rigidity factor measured for a normal RBC is 0.2 compared with 0.5 for RBC pre-treated with GA.

The right hand graph of Fig. 12 shows a continuation of the experiment, where GA is added to the already coupled RBC of the second and third samples. As a result, the rigidity factor increase to a steady state rigidity factor of 0.52, thereby confirming the GA as the cause of the increase rigidity.

The examples above demonstrate the invention's applicability in the field of blood coagulation analysis. However, the invention may be general relevant to biomedical diagnostics, and not only the area of haematology. In fact, the methodology of the invention may find application in any other area where the measurement of dynamic changes fluid viscoelastic properties are required. For example, the invention may find use in industrial processes where the performance of a viscoelastic fluid is critical, such as lubricating oils. Furthermore, the invention may be used in the food and beverage sector e.g. to critically control the viscoelastic properties of foodstuffs (e.g. dairy products such as milk, yogurts, cheeses, etc.) to maintain consistency and consumer experience.

Within the area of haematology, analysis of the viscoelastic properties of blood and blood components can be used for the purpose of identifying a range of disorders and monitoring the effectiveness of various treatments. Examples include:

Monitoring blood clotting time using various clotting time tests such as activated partial thromboplastic time (aPTT), prothrombin time (PT), activated clotting time (ACT), thrombin time, measurement of fibrinogen concentration and/or activity, measurement of d-dimer, fibrin(ogen) degradation products and other clotting factors etc. Assessing the effect of various treatments and drug therapies on these values such as treatment with heparin, low molecular weight heparins, coumarins, direct thrombin inhibitors, Xa inhibitors, etc. Identifying coagulation disorders and clotting factor deficiencies which result in changes in clotting time.

Measuring changes in blood viscoelasticity due to quantitative or qualitative disorders of fibrinogen function.

Measuring changes in behaviour of red blood cells such as red cell deformability and red cell numbers (haematocrit). The former is critical in a number of associated disease conditions such as diabetes and associated angiopathy and retinopathy.

Measuring the behaviour of platelets under a variety of sheer conditions which may represent either venous of arterial conditions. Such would allow the determination of the contribution of platelets to clot formation in VTE or bleeding and whether it was a contributory risk factor, e.g., sticky platelet syndrome, thrombocytopaenia, etc.

Identifying distinct patterns of viscoelastic behaviour, as determined by the novel parameters of $M_{\tilde{i}}$ and $\mu_{\tilde{i}}$ and rigidity factor $\varphi$ that assist in the determination of specific pathophysiological conditions, including those listed above, as well as others such as thrombophilic factors such as Factor V Leiden and disorders of Protein C activity and Protein S, disseminated intravascular coagulation and venous thromboembolism (VTE) and associated deep vein thrombosis (DVT) and pulmonary embolism (PE).

Identification of distinct patterns of viscoelastic behaviour, as determined by these novel parameters that allow the identification of pathophysiological conditions which bring about VTE, but have, as yet, indeterminate cause.

Other biomedical diagnostic uses may be found in any circumstance where the measurement of a viscoelastic fluid may be important in the diagnosis or treatment of diseases and disorders, such as sputum (e.g., cystic fibrosis, tuberculosis and other pulmonary infections and disorders), semen and associated issues of fertility, infection and inflammation, urine, saliva, tears, sweat, etc.

REFERENCES

[1] EP 2 093 556 A2

[2] Cooper MA, Singleton VT. A survey of the 2001 to 2005 quartz crystal microbalance biosensor literature: applications of acoustic physics to the analysis of biomolecular interactions. J Mol Recognit. 2007;20:154-184.

[3] Yakhno T, Sanin A, Pelyushenko A, Kazakov V, Shaposhnikova O, Chernov A, Yakhno V, Vacca C, Falcone F, Johnson B. Uncoated quartz resonator as a universal biosensor. Biosens. Bioelectron. 2007;22:2127-2131.

[4] Kanazawa KK, Gordon JG. Frequency of a quartz microbalance in contact with liquid. Anal. Chem. 1985;57:1770-1771.

[5] Bandey HL, Martin SJ, Cernosek RW. Modeling the Responses of Thickness-Shear Mode Resonators under Various Loading Conditions. Anal. Chem. 1999;71:2205-2214.

[6] Johannsmann D. Viscoelastic, mechanical, and dielectric measurements on complex samples with the quartz crystal microbalance. Phys. Chem. Chem. Phys. 2008;10(31):4516-34.

[7] Johannsmann D. Modeling of QCM Data. http://www.pc.tu-clausthal.de/fileadmin/homes/johannsmann/div/QCM Modeling Tuto rial.pdf

[8] Santos-Martinez MJ, Inkielewicz-Stepniak I, Medina C, Rahme K, D'Arcy KM, Fox D, Holmes JD, Zhang H, Radomski MW. The use of quartz crystal microbalance with dissipation (QCM-D) for studying nanoparticle-induced platelet aggregation. Int. J. of Nanomedicine. 2012;7:243-255.

[9] Reiss B, Janshoff A, Steinem C, Seebach J, Wegener J. Adhesion Kinetics of Functionalized Vesicles and Mammalian Cells: A Comparative Study. Langmuir 2003;19:1816-1823.

[10] Guhr G, Kunze R, Martin G, Schmidt H, Weihnacht M, Gehrisch S, Siegert G. Thrombelastography using acoustic sensors. IEEE Ultrasonics Symposium Proc. 2006:544-547.

[11] Sauerbrey G. Verwendung von schwingquarzen zur wagung dunner schichten und zur mikrowagung. Zeitschrift fur Physik 1959;155:206-222.

[12] Bandey HL, Cernosek RW, Lee WE, Ondrovic LE. Blood rheological characterization using the thickness-shear mode resonator. Biosens. Bioelectron. 2004;19:1657-1665.

[13] Müller L, Sinn S, Drechsel H, Ziegler C, Wendel HP, Northoff H, Gehring FK. Investigation of Prothrombin Time in Human Whole-Blood Samples with a Quartz Crystal Biosensor. Anal. Chem. 2010;82:658-663.

[14] Voinova MV, Jonson M, Kasemo B. Missing mass effect in biosensor's QCM applications. Biosens. Bioelectron. 2002;17:835-41.

[15] Borovikov AP. Measurement of viscosity of media by means of shear vibration of plane piezoresonators. Instruments and Experimental Techniques 1976;19:223-224.

[16] Garg, A., Heflin, J.R., Gibson, H.W., Davis, R.M., 2008. Study of Film Structure and Adsorption Kinetics of Polyelectrolyte Multilayer Films: Effect of pH and Polymer Concentration. Langmuir, 24, 10887-10894.

[17] Piechocka IK, Bacabac RG, Potters M, MacKintosh FC, Koenderink,GH. Structural Hierarchy Governs Fibrin Gel Mechanics. Biophys J. 2010;98:2281-2289.

[18] Ryan EA, Mockros LF, Weisel JW, Lorand L. Structural origins of fibrin clot rheology. Biophys J. 1999;77:2813-2826.

[19] Thurston GB. Non-Newtonian viscosity of human blood: Flow-induced changes in microstructure. Biorheology 1994;31:179-192.

[20] Evans-Nguyen KM, Schoenfischet MH. Fibrin Proliferation at Model Surfaces: Influence of Surface Properties. Langmuir 2005;21:1691-1694.

[21] Wertz CF, Santore MM. Adsorption and Relaxation Kinetics of Albumin and Fibrinogen on Hydrophobic Surfaces: Single-Species and Competitive Behavior. Langmuir 1999;15:8884-8894.

[22] Baker S, Sigley J, Carlisle CR, Stitzel J, Berry J, Bonin K, Guthold M. The Mechanical Properties of Dry, Electrospun Fibrinogen Fibers. Mater Sci Eng. 2012;1,32:215-221.

[23] Falvo MR, Gorkun OV, Lord ST. The molecular origins of the mechanical properties of fibrin, Biophys Chem. 2010;152:15-20.

[23] Maciaszek JL, Lykotrafitis G. Sickle cell trait erythrocytes are significantly stiffer than normal. J Biomech. 2011;44:657-661.

[24] Korin N, Bransky A, Dinnar U. Theoretical model and experimental study of red blood cell (RBC) deformation in microchannels, J Biomech. 2007;40:2088-2095.

[25] Malrnsten M. Ellipsometry studies of the effects of surface hydrophobicity on protein adsorption. Colloids and Surfaces B: Biointerfaces 1995;3:297-308.

[26] Késmárky G, Kenyeres P, Rábai M, Tóth K. Plasma viscosity: A forgotten variable. Clinical Hemorheology and Microcirculation 2008;39:243-246.

[27] Vikinge TP, Hansson KM, Sandstrom P, Liedberg B, Lindahl TL, Lundstrom I, Tengvall P, Hook F. Comparison of surface plasmon resonance and quartz crystal microbalance in the study of whole blood and plasma coagulation. Biosens. Bioelectron. 2000;15:605-613.

**Claims**

1. A method of evaluating viscoelastic properties of a fluid film as it interacts with a surface, the method comprising:

   applying a fluid under test to an oscillating surface of a mechanical resonator sensor, wherein the fluid comprises particles, which, after the fluid under test is applied to the oscillating surface, begin to couple to the oscillating surface to form a film that interacts with the oscillating surface; and
   detecting a change in resonant frequency $\Delta f$ and a change in half bandwidth $\Delta\Gamma$ of a resonance peak exhibited by the mechanical resonance sensor as the fluid under test forms the film that interacts with the oscillating surface; and evaluating two or more of:

   a first quantitative characteristic indicative of an effective mass $M_{\widetilde{t}}$ of the film interacting with the oscillating surface,
   a second quantitative characteristic indicative of an effective elasticity $\mu_{\widetilde{t}}$ of the film interacting with the oscillating surface, and
   a qualitative characteristic indicative of a rigidity factor $\varphi$ of an individual particle within the film interacting with the oscillating surface, wherein:

$$M_{\widetilde{t}} = B\left[\Delta f(t_z) - \Delta f(t) - \frac{\Delta\Gamma(t_z)^2}{\Delta\Gamma(t)} + \Delta\Gamma(t_z)\right]$$

$$\mu_{\widetilde{t}} = A\left[\frac{\Delta\Gamma(t)^2}{\Delta\Gamma(t_z)^2} - \frac{\Delta\Gamma(t_z)^2}{\Delta\Gamma(t)^2}\right]$$

   and

$$\varphi = C\,\frac{\mu_{\widetilde{t}}}{M_{\widetilde{t}}}$$

   where $A$, $B$ and $C$ are coefficients of proportionality, $\Delta f(t)$ and $\Delta\Gamma(t)$ are respectively the detected change in resonant frequency and half bandwidth of the resonant peak at a detection time $t$ when the film has formed and is interacting with the oscillating surface, and $\Delta f(t_z)$ and $\Delta\Gamma(t_z)$ are respectively the detected change in resonant frequency and half bandwidth of the resonant peak at a pre-interaction time $t_z$ which is after the fluid is applied but before any particles couple to the oscillating surface, where $t_z < t$.

2. A method according to claim 1, wherein the film adheres to the surface, and the pre-interaction time $t_z$ occurs at a time before adhesion begins.

3. A method according to claim 1 or 2 including outputting a result of evaluating one or more of the first quantitative characteristic, the second quantitative characteristic and the qualitative characteristic.

4. A method according to claim 3, wherein the result is time-series data for one or more of the first quantitative characteristic, the second quantitative characteristic and the qualitative characteristic.

5. A method according to claim 4 including displaying the time-series data in a graphical manner.

6. A method according to any preceding claim including comparing a result of evaluating one or more of the first quantitative characteristic, the second quantitative characteristic and the qualitative characteristic with a threshold value to classify the fluid under test.

7. A method according to any preceding claim, wherein the changes in resonant frequency $\Delta f$ and half bandwidth $\Delta\Gamma$ of the resonance peak are calculated relative to a resonant frequency $f_0$ and half bandwidth $\Gamma_0$ of the mechanical resonator sensor before the fluid is applied to the oscillating surface.

8. A method according to any preceding claim, wherein the fluid behaves as a Newtonian fluid at the pre-interaction time $t_z$.

9. A method according to any preceding claim, wherein detecting a change in half bandwidth $\Delta\Gamma$ includes determining a dissipation value $D$ or a Q-factor value $Q$.

10. A method according to any preceding claim, wherein the fluid under test is blood or a blood component.

11. A method according to claim 10 including identifying a pattern in one or more of the first quantitative characteristic, the second quantitative characteristic and the qualitative characteristic, and determining a pathological condition based on the identified pattern.

12. A method according to claim 10 including determining information indicative of one or more of:

blood clotting time,
red blood cell deformability,
platelet aggregability,
erythrocyte sedimentation,
thrombotic risk, and
fibrinogen activity,

based on one or more of the first quantitative characteristic, the second quantitative characteristic and the qualitative characteristic.

13. A computer program product comprising instructions stored on a computer-readable medium that are executable by a computer to perform a method comprising:

receiving input data comprising a change in resonant frequency $\Delta f$ and a change in half bandwidth $\Delta\Gamma$ of a resonance peak exhibited by a mechanical resonance sensor as a fluid under test forms a film interacting with an oscillating surface of the mechanical resonance sensor, wherein the fluid comprises particles, which, after the fluid under test is applied to the oscillating surface, begin to couple to the oscillating surface to form the film that interacts with the oscillating surface; and evaluating two or more of:

a first quantitative characteristic indicative of an effective mass $M_{\tilde{t}}$ of the film interacting with the oscillating surface,
a second quantitative characteristic indicative of an effective elasticity $\mu_{\tilde{t}}$ of the film interacting with the oscillating surface, and
a qualitative characteristic indicative of a rigidity factor $\varphi$ of an individual particle within the film interacting with the oscillating surface; and

outputting the first quantitative characteristic, the second quantitative characteristic or the qualitative characteristic,
wherein:

$$M_{\tilde{t}} = B\left[\Delta f(t_z) - \Delta f(t) - \frac{\Delta\Gamma(t_z)^2}{\Delta\Gamma(t)} + \Delta\Gamma(t_z)\right]$$

$$\mu_{\tilde{t}} = A\left[\frac{\Delta\Gamma(t)^2}{\Delta\Gamma(t_z)^2} - \frac{\Delta\Gamma(t_z)^2}{\Delta\Gamma(t)^2}\right]$$

and

$$\varphi = C\frac{\mu_{\tilde{t}}}{M_{\tilde{t}}}$$

where *A, B* and *C* are coefficients of proportionality, $\Delta f(t)$ and $\Delta\Gamma(t)$ are respectively the detected change in resonant frequency and half bandwidth of the resonant peak at a detection time *t* when the film has formed and is interacting with the oscillating surface, and Δf(*t_z*) and ΔΓ(*t_z*) are respectively the detected change in resonant frequency and half bandwidth of the resonant peak at a pre-interaction time *t_z* which is after the fluid is applied but before any particles couple to the oscillating surface, where *t_z*<*t*.

14. An apparatus for evaluating viscoelastic properties of a fluid film as it interacts with a surface, the apparatus comprising:

a mechanical resonator sensor having an oscillatable surface for receiving a fluid under test, wherein the fluid comprises particles, which, after the fluid under test is applied to the oscillatable surface, begin to couple to the oscillatable surface to form a film that interacts with the oscillatable surface; and

a processing device in communication with the mechanical resonator sensor receive therefrom a change in resonant frequency Δ*f* and a change in half bandwidth ΔΓ of a resonance peak exhibited by the mechanical resonance sensor as the fluid under test forms the film interacting with the oscillatable surface,

wherein the processing device is arranged to calculate output data representative of properties of the fluid under test, and the output data includes two or more of:

a first quantitative characteristic indicative of an effective mass $M_{\tilde{t}}$ of the film interacting with the oscillatable surface,

a second quantitative characteristic indicative of an effective elasticity $\mu_{\tilde{t}}$ of the film interacting with the oscillatable surface, and

a qualitative characteristic indicative of a rigidity factor of an individual particle within the film interacting with the oscillatable surface,

wherein:

$$M_{\tilde{t}} = B\left[\Delta f(t_z) - \Delta f(t) - \frac{\Delta\Gamma(t_z)^2}{\Delta\Gamma(t)} + \Delta\Gamma(t_z)\right]$$

$$\mu_{\tilde{t}} = A\left[\frac{\Delta\Gamma(t)^2}{\Delta\Gamma(t_z)^2} - \frac{\Delta\Gamma(t_z)^2}{\Delta\Gamma(t)^2}\right]$$

and

$$\varphi = C\frac{\mu_{\tilde{t}}}{M_{\tilde{t}}}$$

where *A, B* and *C* are coefficients of proportionality, Δ*f*(*t*) and ΔΓ(*t*) are respectively the detected change in resonant frequency and half bandwidth of the resonant peak at a detection time *t* when the film has formed and is interacting with the oscillating surface, and Δf(*t_z*) and ΔΓ(*t_z*) are respectively the detected change in resonant frequency and half bandwidth of the resonant peak at a pre-interaction time *t_z* which is after the fluid is applied but before any particles couple to the oscillating surface, where *t_z*<*t*.

15. An apparatus according to claim 14, wherein the mechanical resonator sensor comprises a piezoelectric resonator.

16. An apparatus according to claim 14, wherein the mechanical resonator sensor comprises a quartz crystal microbalance.

**Patentansprüche**

1. Verfahren zur Evaluierung von viskoelastischen Eigenschaften eines Fluidfilms bei Interagieren desselben mit einer Oberfläche, wobei das Verfahren Folgendes umfasst:

Auftragen eines zu testenden Fluids auf eine Oszillationsfläche eines mechanischen Resonatorsensors, worin das Fluid Partikel umfasst, die sich nach Auftragen des zu testenden Fluids auf die Oszillationsfläche mit der Oszillationsfläche zu koppeln beginnen, um einen Film zu bilden, der mit der Oszillationsfläche in Wechselwirkung ist; und

Detektieren einer Änderung in einer Resonanzfrequenz $\Delta f$ und einer Änderung in einer halben Bandbreite $\Delta\Gamma$ einer durch den mechanischen Resonanzsensor aufgezeigten Resonanzspitze, wenn das zu testende Fluid den Film bildet, der mit der Oszillationsfläche in Wechselwirkung ist; und

Evaluieren von zwei oder mehreren von Folgendem:

eine erste quantitative Eigenschaft, die auf eine effektive Masse $M_{\tilde{t}}$ des mit der Oszillationsfläche interagierenden Films hinweist,

eine zweite quantitative Eigenschaft, die auf eine effektive Elastizität $\mu_{\tilde{t}}$ des mit der Oszillationsfläche interagierenden Films hinweist, und

eine qualitative Eigenschaft, die auf einen Steifigkeitsfaktor $\varphi$ eines jeweiligen Partikels innerhalb des mit der Oszillationsfläche interagierenden Films hinweist,

worin:

$$M_{\tilde{t}} = B\left[\Delta f(t_z) - \Delta f(t) - \frac{\Delta\Gamma(t_z)^2}{\Delta\Gamma(t)} + \Delta\Gamma(t_z)\right]$$

$$\mu_{\tilde{t}} = A\left[\frac{\Delta\Gamma(t)^2}{\Delta\Gamma(t_z)^2} - \frac{\Delta\Gamma(t_z)^2}{\Delta\Gamma(t)^2}\right]$$

und

$$\varphi = C\frac{\mu_{\tilde{t}}}{M_{\tilde{t}}}$$

worin A, B und C Proportionalitätskoeffizienten sind, $\Delta f(t)$ und $\Delta\Gamma(t)$ jeweils die detektierte Änderung in der Resonanzfrequenz und in der halben Bandbreite der Resonanzspitze bei einer Detektionszeit t darstellen, wenn der Film sich ausgebildet hat und mit der Oszillationsfläche in Wechselwirkung ist, und $\Delta f(t_Z)$ und $\Delta\Gamma(f_Z)$ jeweils die detektierte Änderung in der Resonanzfrequenz und in der halben Bandbreite der Resonanzspitze bei einer vorbestimmten Zeit $t_Z$ darstellen, die nach Auftragen des Fluids, aber vor einem Koppeln von Partikeln mit der Oszillationsfläche erfolgt, worin $t_Z < t$.

2. Verfahren gemäß Anspruch 1, worin der Film an der Oberfläche haftet und die Vor-Interaktionszeit $t_Z$ zu einem Zeitpunkt vor Beginn der Haftung erfolgt.

3. Verfahren gemäß Anspruch 1 oder 2, umfassend das Ausgeben eines Ergebnisses des Evaluierens einer oder mehrerer der ersten quantitativen Eigenschaft, der zweiten quantitativen Eigenschaft und der qualitativen Eigenschaft.

4. Verfahren gemäß Anspruch 3, worin das Ergebnis Zeitreihendaten für eine oder mehrere der ersten quantitativen Eigenschaft, der zweiten quantitativen Eigenschaft und der qualitativen Eigenschaft darstellt.

5. Verfahren gemäß Anspruch 4, umfassend das Anzeigen der Zeitreihendaten in graphischer Art und Weise.

6. Verfahren gemäß einem der vorangegangenen Ansprüche, umfassend das Vergleichen eines Ergebnisses des Evaluierens einer oder mehrerer der ersten quantitativen Eigenschaft, der zweiten quantitativen Eigenschaft und der qualitativen Eigenschaft mit einem Schwellenwert, um das zu testende Fluid zu klassifizieren.

7. Verfahren gemäß einem der vorangegangenen Ansprüche, worin die Änderungen in der Resonanzfrequenz $\Delta f$ und in der halben Bandbreite $\Delta\Gamma$ der Resonanzspitze relativ zu einer Resonanzfrequenz $f_0$ und der halben Bandbreite $\Gamma_0$ des mechanischen Resonatorsensors vor Auftragen des Fluids auf die Oszillationsfläche berechnet werden.

8. Verfahren gemäß einem der vorangegangenen Ansprüche, worin sich das Fluid wie ein Newtonsches Fluid bei der Vor-Interaktionszeit $t_Z$ verhält.

9. Verfahren gemäß einem der vorangegangenen Ansprüche, worin das Detektieren einer Änderung in der halben Bandbreite $\Delta\Gamma$ das Bestimmen eines Dissipationswerts D oder eines Q-Faktor-Werts Q umfasst.

10. Verfahren gemäß einem der vorangegangenen Ansprüche, worin das zu testende Fluid Blut oder eine Blutkomponente ist.

11. Verfahren gemäß Anspruch 10, umfassend das Identifizieren einer Struktur in einer oder mehreren der ersten quantitativen Eigenschaft, der zweiten quantitativen Eigenschaft und der qualitativen Eigenschaft und das Bestimmen eines pathologischen Zustands auf Basis der identifizierten Struktur.

12. Verfahren gemäß Anspruch 10, umfassend das Bestimmen von Informationen, die auf einen oder mehrere Faktoren von Folgendem hinweisen:

> Blutgerinnungszeit,
> Verformbarkeit von roten Blutkörperchen,
> Aggregierfähigkeit von Blutplättchen,
> Erythrozytensedimentation,
> Thromboserisiko und
> Fibronogen-Aktivität,

und zwar auf Basis einer oder mehrerer der ersten quantitativen Eigenschaft, der zweiten quantitativen Eigenschaft und der qualitativen Eigenschaft.

13. Computerprogrammprodukt, das auf einem computerlesbaren Medium gespeicherte Befehle umfasst, die durch einen Computer ausführbar sind, um ein Verfahren auszuführen, das Folgendes umfasst:

> Empfangen von Eingangsdaten, die eine Änderung in der Resonanzfrequenz $\Delta f$ und eine Änderung in der halben Bandbreite $\Delta\Gamma$ einer durch einen mechanischen Resonanzsensor aufgezeigten Resonanzspitze umfassen, wenn ein zu testendes Fluid einen Film bildet, der mit einer Oszillationsfläche des mechanischen Resonanzsensors in Wechselwirkung ist, worin das Fluid Partikel umfasst, die nach Auftragen des zu testenden Fluids auf die Oszillationsfläche sich mit der Oszillationsfläche zu koppeln beginnen, um den Film zu bilden, der mit der Oszillationsfläche in Wechselwirkung ist; und
> Evaluieren von zwei oder mehreren von Folgendem:

>> eine erste quantitative Eigenschaft, die auf eine effektive Masse $M_{\tilde{i}}$ des mit der Oszillationsfläche interagierenden Films hinweist,
>> eine zweite quantitative Eigenschaft, die auf eine effektive Elastizität $\mu_{\tilde{i}}$ des mit der Oszillationsfläche interagierenden Films hinweist, und
>> eine qualitative Eigenschaft, die auf einen Steifigkeitsfaktor $\varphi$ eines jeweiligen Partikels innerhalb des mit der Oszillationsfläche interagierenden Films hinweist; und

> Ausgeben der ersten quantitativen Eigenschaft, der zweiten quantitativen Eigenschaft oder der qualitativen Eigenschaft,
> worin:

$$M_{\tilde{i}} = B\left[\Delta f(t_z) - \Delta f(t) - \frac{\Delta\Gamma(t_z)^2}{\Delta\Gamma(t)} + \Delta\Gamma(t_z)\right]$$

$$\mu_{\tilde{i}} = A\left[\frac{\Delta\Gamma(t)^2}{\Delta\Gamma(t_z)^2} - \frac{\Delta\Gamma(t_z)^2}{\Delta\Gamma(t)^2}\right]$$

und

$$\varphi = C \frac{\tilde{\mu_t}}{\tilde{M_t}}$$

worin A, B und C Proportionalitätskoeffizienten sind, $\Delta f(t)$ und $\Delta\Gamma(t)$ jeweils die detektierte Änderung in der Resonanzfrequenz und in der halben Bandbreite der Resonanzspitze bei einer Detektionszeit t darstellen, wenn der Film sich ausgebildet hat und mit der Oszillationsfläche in Wechselwirkung ist, und $\Delta f(t_Z)$ und $\Delta\Gamma(t_Z)$ jeweils die detektierte Änderung in der Resonanzfrequenz und in der halben Bandbreite der Resonanzspitze bei einer Vor-Interaktionszeit $t_Z$ darstellen, die nach Auftragen des Fluids, aber vor einem Koppeln von Partikeln mit der Oszillationsfläche erfolgt, worin $t_Z < t$.

14. Vorrichtung zur Evaluierung von viskoelastischen Eigenschaften eines Fluidfilms bei Interagieren desselben mit einer Oberfläche, wobei die Vorrichtung Folgendes umfasst:

einen mechanischen Resonatorsensor, der eine oszillierbare Oberfläche zur Aufnahme eines zu testenden Fluids aufweist, worin das Fluid Partikel umfasst, die nach Auftragen des zu testenden Fluids auf die oszillierbare Oberfläche mit der oszillierbaren Oberfläche sich zu koppeln beginnen, um einen Film zu bilden, der in Wechselwirkung mit der oszillierbaren Oberfläche ist; und
eine Verarbeitungsvorrichtung, die in Kommunikation mit dem mechanischen Resonatorsensor ist, um von diesem eine Änderung in der Resonanzfrequenz $\Delta f$ und eine Änderung in der halben Bandbreite $\Delta\Gamma$ einer durch den mechanischen Resonatorsensor aufgezeigten Resonanzspitze zu empfangen, wenn das zu testende Fluid den mit der oszillierbaren Oberfläche interagierenden Film bildet,
worin die Verarbeitungseinheit ausgelegt ist, um Ausgangsdaten, die für Eigenschaften des zu testenden Fluids repräsentativ sind, zu berechnen, und worin die Ausgangsdaten zwei oder mehrere von Folgendem umfassen:

eine erste quantitative Eigenschaft, die auf eine effektive Masse $M_{\tilde{t}}$ des mit der oszillierbaren Oberfläche interagierenden Films hinweist,
eine zweite quantitative Eigenschaft, die auf eine effektive Elastizität $\mu_{\tilde{t}}$ des mit der oszillierbaren Oberfläche interagierenden Films hinweist, und
eine qualitative Eigenschaft, die auf einen Steifigkeitsfaktor eines jeweiligen Partikels innerhalb des mit der oszillierbaren Oberfläche interagierenden Films hinweist;

worin:

$$\tilde{M_t} = B\left[\Delta f(t_z) - \Delta f(t) - \frac{\Delta\Gamma(t_z)^2}{\Delta\Gamma(t)} + \Delta\Gamma(t_z)\right]$$

$$\tilde{\mu_t} = A\left[\frac{\Delta\Gamma(t)^2}{\Delta\Gamma(t_z)^2} - \frac{\Delta\Gamma(t_z)^2}{\Delta\Gamma(t)^2}\right]$$

und

$$\varphi = C \frac{\tilde{\mu_t}}{\tilde{M_t}}$$

worin A, B und C Proportionalitätskoeffizienten sind, $\Delta f(t)$ und $\Delta\Gamma(t)$ jeweils die detektierte Änderung in der Resonanzfrequenz und in der halben Bandbreite der Resonanzspitze bei einer Detektionszeit t darstellen, wenn der Film sich ausgebildet hat und mit der Oszillationsfläche in Wechselwirkung ist, und $\Delta f(t_Z)$ und $\Delta\Gamma(t_Z)$ jeweils die detektierte Änderung in der Resonanzfrequenz und in der halben Bandbreite der Resonanzspitze bei einer Vor-Interaktionszeit $t_Z$ darstellen, die nach Auftragen des Fluids, jedoch vor einem Koppeln von Partikeln mit der Oszillationsfläche erfolgt, worin $t_Z < t$.

15. Vorrichtung gemäß Anspruch 14, worin der mechanische Resonatorsensor einen piezoelektrischen Resonator umfasst.

**16.** Vorrichtung gemäß Anspruch 14, worin der mechanische Resonatorsensor eine Quarzkristall-Mikrowaage umfasst.

**Revendications**

**1.** Procédé d'évaluation des propriétés viscoélastiques d'un film de fluide lorsqu'il interagit avec une surface, le procédé comprenant :

l'application d'un fluide testé sur une surface oscillante d'un capteur de résonateur mécanique, dans lequel le fluide comprend des particules, qui, une fois que le fluide testé a été appliqué sur la surface oscillante, commence à se coupler avec la surface oscillante afin de former un film qui interagit avec la surface oscillante ; et la détection d'un changement de fréquence de résonance $\Delta f$ et d'un changement de demi-largeur de bande $\Delta\Gamma$ d'un pic de résonance présenté par le capteur de résonance mécanique lorsque le fluide testé forme le film qui interagit avec la surface oscillante ;
et l'évaluation de deux ou plus de ce qui suit :

une première caractéristique quantitative qui indique une masse effective $M\tilde{~}_t$ du film qui interagit avec la surface oscillante,
une seconde caractéristique quantitative qui indique une élasticité effective $\mu\tilde{~}_t$ du film qui interagit avec la surface oscillante, et
une caractéristique qualitative qui indique un facteur de rigidité $\varphi$ d'une particule individuelle dans le film qui interagit avec la surface oscillante, dans lequel :

$$\tilde{M_t} = B\left[\Delta f(t_z) - \Delta f(t) - \frac{\Delta\Gamma(t_z)^2}{\Delta\Gamma(t)} + \Delta\Gamma(t_z)\right]$$

$$\tilde{\mu_t} = A\left[\frac{\Delta\Gamma(t)^2}{\Delta\Gamma(t_z)^2} - \frac{\Delta\Gamma(t_z)^2}{\Delta\Gamma(t)^2}\right]$$

et

$$\varphi = C\frac{\tilde{\mu_t}}{\tilde{M_t}}$$

où $A$, $B$ et $C$ sont des coefficients de proportionnalité, $\Delta f(t)$ et $\Delta\Gamma(t)$ sont respectivement le changement détecté de fréquence de résonance et de demi-largeur de bande du pic de résonance à un moment de détection t lorsque le film s'est formé et interagit avec la surface oscillante, et $\Delta f(t_z)$ et $\Delta\Gamma(t_z)$ sont respectivement le changement détecté de fréquence de résonance et de demi-largeur de bande du pic de résonance à un moment de pré-interaction $t_z$ qui est postérieur au moment auquel le fluide est appliqué mais antérieur au moment auquel les particules se couplent à la surface oscillante, où $t_z < t$.

**2.** Procédé selon la revendication 1, dans lequel le film adhère à la surface, et le moment de pré-interaction $t_z$ a lieu à un moment antérieur au début de l'adhérence.

**3.** Procédé selon la revendication 1 ou 2, comprenant la délivrance d'un résultat d'évaluation d'une ou plusieurs de la première caractéristique quantitative, de la seconde caractéristique quantitative et de la caractéristique qualitative.

**4.** Procédé selon la revendication 3, dans lequel le résultat est des données de série temporelle pour une ou plusieurs de la première caractéristique quantitative, de la seconde caractéristique quantitative et de la caractéristique qualitative.

**5.** Procédé selon la revendication 4, comprenant l'affichage des données de série temporelle de manière graphique.

**6.** Procédé selon l'une quelconque des revendications précédentes, comprenant la comparaison d'un résultat d'évaluation d'une ou plusieurs de la première caractéristique quantitative, de la seconde caractéristique quantitative et de la caractéristique qualitative avec une valeur de seuil afin de classifier le fluide testé.

7. Procédé selon l'une quelconque des revendications précédentes, dans lequel les changements de fréquence de résonance $\Delta f$ et de demi-largeur de bande $\Delta\Gamma$ du pic de résonance sont calculés par rapport à une fréquence de résonance $f_0$ et une demi-largeur de bande $\Gamma_0$ du capteur de résonateur mécanique avant que le fluide soit appliqué sur la surface oscillante.

8. Procédé selon l'une quelconque des revendications précédentes, dans lequel le fluide se comporte comme un fluide newtonien au moment de pré-interaction $t_z$.

9. Procédé selon l'une quelconque des revendications précédentes, dans lequel la détection d'un changement de demi-largeur de bande $\Delta\Gamma$ comprend la détermination d'une valeur de dissipation $D$ ou d'une valeur de facteur Q $Q$.

10. Procédé selon l'une quelconque des revendications précédentes, dans lequel le fluide testé est du sang ou un composant sanguin.

11. Procédé selon la revendication 10, comprenant l'identification d'un modèle dans une ou plusieurs de la première caractéristique quantitative, de la seconde caractéristique quantitative et de la caractéristique qualitative, et la détermination d'une condition pathologique sur la base du modèle identifié.

12. Procédé selon la revendication 10, comprenant la détermination d'informations qui indiquent un ou plusieurs de ce qui suit :

le temps de coagulation du sang,
la déformabilité des globules rouges,
la capacité d'agrégation plaquettaire,
la sédimentation des hématies,
le risque thrombotique, et
l'activité fibrinogène,
sur la base d'une ou plusieurs de la première caractéristique quantitative, de la seconde caractéristique quantitative et de la caractéristique qualitative.

13. Produit de programme informatique comprenant des instructions stockées sur un support lisible par un ordinateur qui peuvent être exécutées par un ordinateur afin d'exécuter un procédé comprenant :

la réception de données d'entrée comprenant un changement de fréquence de résonance $\Delta f$ et de demi-largeur de bande $\Delta\Gamma$ d'un pic de résonance présenté par un capteur de résonance mécanique lorsqu'un fluide testé forme un film qui interagit avec une surface oscillante du capteur de résonance mécanique, dans lequel le fluide comprend des particules, qui, une fois que le fluide testé a été appliqué sur la surface oscillante, commence à se coupler avec la surface oscillante afin de former le film qui interagit avec la surface oscillante ;
et l'évaluation de deux ou plus de ce qui suit :

une première caractéristique quantitative qui indique une masse effective $M\!\sim_t$ du film qui interagit avec la surface oscillante,

une seconde caractéristique quantitative qui indique une élasticité effective $\mu\!\sim_t$ du film qui interagit avec la surface oscillante, et
une caractéristique qualitative qui indique un facteur de rigidité $\varphi$ d'une particule individuelle dans le film qui interagit avec la surface oscillante ; et

la délivrance de la première caractéristique quantitative, de la seconde caractéristique quantitative ou de la caractéristique qualitative,

dans lequel :

$$M_t^{\sim} = B \left[ \Delta f(t_z) - \Delta f(t) - \frac{\Delta \Gamma(t_z)^2}{\Delta \Gamma(t)} + \Delta \Gamma(t_z) \right]$$

$$\mu_t^{\sim} = A \left[ \frac{\Delta \Gamma(t)^2}{\Delta \Gamma(t_z)^2} - \frac{\Delta \Gamma(t_z)^2}{\Delta \Gamma(t)^2} \right]$$

et

$$\varphi = C \frac{\mu_t^{\sim}}{M_t^{\sim}}$$

où *A, B* et *C* sont des coefficients de proportionnalité, $\Delta f(t)$ et $\Delta \Gamma(t)$ sont respectivement le changement détecté de fréquence de résonance et de demi-largeur de bande du pic de résonance à un moment de détection *t* lorsque le film s'est formé et interagit avec la surface oscillante, et $\Delta f(t_z)$ et $\Delta \Gamma(t_z)$ sont respectivement le changement détecté de fréquence de résonance et de demi-largeur de bande du pic de résonance à un moment de pré-interaction $t_z$ qui est postérieur au moment auquel le fluide est appliqué mais antérieur au moment auquel les particules se couplent à la surface oscillante, où $t_z < t$.

14. Appareil d'évaluation des propriétés viscoélastiques d'un film de fluide lorsqu'il interagit avec une surface, l'appareil comprenant :

un capteur de résonateur mécanique ayant une surface oscillante destinée à recevoir un fluide testé, dans lequel le fluide comprend des particules, qui, une fois que le fluide testé a été appliqué sur la surface oscillante, commencent à se coupler avec la surface oscillante afin de former un film qui interagit avec la surface oscillante ; et

un dispositif de traitement en communication avec le capteur de résonateur mécanique afin de recevoir de la part de celui-ci un changement de fréquence de résonance $\Delta f$ et un changement de demi-largeur de bande $\Delta \Gamma$ d'un pic de résonance présenté par le capteur de résonance mécanique lorsque le fluide testé forme le film qui interagit avec la surface oscillante,

dans lequel le dispositif de traitement est prévu pour calculer des données de sortie représentatives des propriétés du fluide testé,

et

les données de sortie comprennent deux ou plus de ce qui suit :

une première caractéristique quantitative qui indique une masse effective $M^{\sim}_t$ du film qui interagit avec la surface oscillante,

une seconde caractéristique quantitative qui indique une élasticité effective $\mu^{\sim}_t$ du film qui interagit avec la surface oscillante, et

une caractéristique qualitative qui indique un facteur de rigidité $\varphi$ d'une particule individuelle dans le film qui interagit avec la surface oscillante,

dans lequel :

$$M_t^{\sim} = B \left[ \Delta f(t_z) - \Delta f(t) - \frac{\Delta \Gamma(t_z)^2}{\Delta \Gamma(t)} + \Delta \Gamma(t_z) \right]$$

$$\mu_t^{\sim} = A \left[ \frac{\Delta \Gamma(t)^2}{\Delta \Gamma(t_z)^2} - \frac{\Delta \Gamma(t_z)^2}{\Delta \Gamma(t)^2} \right]$$

et

$$\varphi = C\,\frac{\tilde{\mu_t}}{\tilde{M_t}}$$

où *A, B* et *C* sont des coefficients de proportionnalité, $\Delta f(t)$ et $\Delta\Gamma(t)$ sont respectivement le changement détecté de fréquence de résonance et de demi-largeur de bande du pic de résonance à un moment de détection *t* lorsque le film s'est formé et interagit avec la surface oscillante, et $\Delta f(t_z)$ et $\Delta\Gamma(t_z)$ sont respectivement le changement détecté de fréquence de résonance et de demi-largeur de bande du pic de résonance à un moment de pré-interaction $t_z$ qui est postérieur au moment auquel le fluide est appliqué mais antérieur au moment auquel les particules se couplent à la surface oscillante, où $t_z < t$.

**15.** Appareil selon la revendication 14, dans lequel le capteur de résonateur mécanique comprend un résonateur piézoélectrique.

**16.** Appareil selon la revendication 14, dans lequel le capteur de résonateur mécanique comprend une microbalance à cristal de quartz.

FIG. 1

FIG. 2

FIG. 3

FIG. 4

FIG. 5

FIG. 6

FIG. 7

FIG. 8

FIG. 9

FIG. 10

FIG. 11

FIG. 12

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- US 4862384 A
- US 20110129929 A
- US 20130211748 A
- EP 2093556 A2

## Non-patent literature cited in the description

- **LAKSHMANAN et al.** Measurement of the evolution of rigid and viscoelastic mass contributions from fibrin network formation during plasma coagulation using quartz crystal microbalance. *Sensors and Actuators B,* vol. 192, 23-28
- **MÜLLER et al.** Investigation of prothrombin time in human whole-blood samples with a quartz crystal biosensor. *Anal. Chem.,* 2010, vol. 82, 658-663
- **KANAZAWA et al.** The oscillation frequency of a quartz resonator in contact with a liquid. *Analytica Chimica Acta,* 1985, vol. 175, 99-105
- **COOPER MA ; SINGLETON VT.** A survey of the 2001 to 2005 quartz crystal microbalance biosensor literature: applications of acoustic physics to the analysis of biomolecular interactions. *J Mol Recognit.,* 2007, vol. 20, 154-184
- **YAKHNO T ; SANIN A ; PELYUSHENKO A ; KAZAKOV V ; SHAPOSHNIKOVA O ; CHERNOV A ; YAKHNO V ; VACCA C ; FALCONE F ; JOHNSON B.** Uncoated quartz resonator as a universal biosensor. *Biosens. Bioelectron.,* 2007, vol. 22, 2127-2131
- **KANAZAWA KK ; GORDON JG.** Frequency of a quartz microbalance in contact with liquid. *Anal. Chem.,* 1985, vol. 57, 1770-1771
- **BANDEY HL ; MARTIN SJ ; CERNOSEK RW.** Modeling the Responses of Thickness-Shear Mode Resonators under Various Loading Conditions. *Anal. Chem.,* 1999, vol. 71, 2205-2214
- **JOHANNSMANN D.** Viscoelastic, mechanical, and dielectric measurements on complex samples with the quartz crystal microbalance. *Phys. Chem. Chem. Phys.,* 2008, vol. 10 (31), 4516-34
- **JOHANNSMANN D.** *Modeling of QCM Data, http://www.pc.tu-clausthal.de/fileadmin/homes/johannsmann/div/QCM Modeling Tuto rial.pdf*
- **SANTOS-MARTINEZ MJ ; INKIELEWICZ-STEPNIAK I ; MEDINA C ; RAHME K ; D'ARCY KM ; FOX D ; HOLMES JD ; ZHANG H ; RADOMSKI MW.** The use of quartz crystal microbalance with dissipation (QCM-D) for studying nanoparticle-induced platelet aggregation. *Int. J. of Nanomedicine,* 2012, vol. 7, 243-255
- **REISS B ; JANSHOFF A ; STEINEM C ; SEEBACH J ; WEGENER J.** Adhesion Kinetics of Functionalized Vesicles and Mammalian Cells: A Comparative Study. *Langmuir,* 2003, vol. 19, 1816-1823
- **GUHR G ; KUNZE R ; MARTIN G ; SCHMIDT H ; WEIHNACHT M ; GEHRISCH S ; SIEGERT G.** Thrombelastography using acoustic sensors. *IEEE Ultrasonics Symposium Proc.,* 2006, 544-547
- **SAUERBREY G.** Verwendung von schwingquarzen zur wagung dunner schichten und zur mikrowagung. *Zeitschrift fur Physik,* 1959, vol. 155, 206-222
- **BANDEY HL ; CERNOSEK RW ; LEE WE ; ONDROVIC LE.** Blood rheological characterization using the thickness-shear mode resonator. *Biosens. Bioelectron,* 2004, vol. 19, 1657-1665
- **MÜLLER L ; SINN S ; DRECHSEL H ; ZIEGLER C ; WENDEL HP ; NORTHOFF H ; GEHRING FK.** Investigation of Prothrombin Time in Human Whole-Blood Samples with a Quartz Crystal Biosensor. *Anal. Chem.,* 2010, vol. 82, 658-663
- **VOINOVA MV ; JONSON M ; KASEMO B.** Missing mass effect in biosensor's QCM applications. *Biosens. Bioelectron,* 2002, vol. 17, 835-41
- **BOROVIKOV AP.** Measurement of viscosity of media by means of shear vibration of plane piezoresonators. *Instruments and Experimental Techniques,* 1976, vol. 19, 223-224
- **GARG, A. ; HEFLIN, J.R. ; GIBSON, H.W. ; DAVIS, R.M.** Study of Film Structure and Adsorption Kinetics of Polyelectrolyte Multilayer Films: Effect of pH and Polymer Concentration. *Langmuir,* 2008, vol. 24, 10887-10894
- **PIECHOCKA IK ; BACABAC RG ; POTTERS M ; MACKINTOSH FC ; KOENDERINK,GH.** Structural Hierarchy Governs Fibrin Gel Mechanics. *Biophys J.,* 2010, vol. 98, 2281-2289
- **RYAN EA ; MOCKROS LF ; WEISEL JW ; LORAND L.** Structural origins of fibrin clot rheology. *Biophys J,* 1999, vol. 77, 2813-2826
- **THURSTON GB.** Non-Newtonian viscosity of human blood: Flow-induced changes in microstructure. *Biorheology,* 1994, vol. 31, 179-192

- **EVANS-NGUYEN KM ; SCHOENFISCHET MH.** Fibrin Proliferation at Model Surfaces: Influence of Surface Properties. *Langmuir,* 2005, vol. 21, 1691-1694
- **WERTZ CF ; SANTORE MM.** Adsorption and Relaxation Kinetics of Albumin and Fibrinogen on Hydrophobic Surfaces: Single-Species and Competitive Behavior. *Langmuir,* 1999, vol. 15, 8884-8894
- **BAKER S ; SIGLEY J ; CARLISLE CR ; STITZEL J ; BERRY J ; BONIN K ; GUTHOLD M.** The Mechanical Properties of Dry, Electrospun Fibrinogen Fibers. *Mater Sci Eng.,* 2012, vol. 1 (32), 215-221
- **FALVO MR ; GORKUN OV ; LORD ST.** The molecular origins of the mechanical properties of fibrin. *Biophys Chem.,* 2010, vol. 152, 15-20
- **MACIASZEK JL ; LYKOTRAFITIS G.** Sickle cell trait erythrocytes are significantly stiffer than normal. *J Biomech.,* 2011, vol. 44, 657-661
- **KORIN N ; BRANSKY A ; DINNAR U.** Theoretical model and experimental study of red blood cell (RBC) deformation in microchannels. *J Biomech.,* 2007, vol. 40, 2088-2095
- **MALRNSTEN M.** Ellipsometry studies of the effects of surface hydrophobicity on protein adsorption. *Colloids and Surfaces B: Biointerfaces,* 1995, vol. 3, 297-308
- **KÉSMÁRKY G ; KENYERES P ; RÁBAI M ; TÓTH K.** Plasma viscosity: A forgotten variable. *Clinical Hemorheology and Microcirculation,* 2008, vol. 39, 243-246
- **VIKINGE TP ; HANSSON KM ; SANDSTROM P ; LIEDBERG B ; LINDAHL TL ; LUNDSTROM I ; TENGVALL P ; HOOK F.** Comparison of surface plasmon resonance and quartz crystal microbalance in the study of whole blood and plasma coagulation. *Biosens. Bioelectron,* 2000, vol. 15, 605-613